# FASCICULE DE BREVET EUROPEEN

(11) **EP 2 459 586 B1**
(45) Date de publication et mention de la délivrance du brevet: **13.08.2014**
(21) Numéro de dépôt: 10737601.4
(22) Date de dépôt: 08.06.2010
(51) Int. Cl.: C07K 14/31, G01N 33/569

(54) **ANTIGENES PROTEIQUES DESTINES AU SERODIAGNOSTIC DES INFECTIONS A STAPHYLOCOCCUS AUREUS NOTAMMENT SUR PROTHESES OSTEO-ARTICULAIRES**
PROTEINANTIGENE FÜR DIE SERODIAGNOSE VON STAPHYLOCOCCUS AUREUS-INFEKTIONEN, IM BESONDEREN AUF OSTEOARTIKULÄREN PROTHESEN
PROTEIN ANTIGENS FOR THE SERODIAGNOSIS OF STAPHYLOCOCCUS AUREUS INFECTIONS, IN PARTICULAR ON OSTEOARTICULAR PROSTHESES

(30) Priorité: 10.06.2009 US 213452 P
(43) Date de publication de la demande: 06.06.2012
(73) Titulaire: InGen Biosciences, 91380 Chilly Mazarin (FR)
(72) Inventeur: CYNCYNATUS, Camille, 92340 Bourg-La-Reine (FR); ROGE, Julie, 92120 Montrouge (FR); THOMAS, Damien, 91380 Chilly Mazarin (FR); NUYTTENS, Hélène, 94200 Ivry sur Seine (FR)
(74) Mandataire: Blot, Philippe Robert Emile
(86) Numéro de dépôt international: PCT/FR2010/051132
(87) Numéro de publication internationale: WO 2010/142906

(56) Documents cités:
- WO-A2-02/094868
- GABRIELLA POCSFALVI ET AL: "Proteomic analysis of exoproteins expressed by enterotoxigenic Staphylococcus aureus strains", PROTEOMICS, WILEY - VCH VERLAG, WEINHEIM, DE LNKD- DOI:10.1002/PMIC.200700965, vol. 8, 1 janvier 2008 (2008-01-01), pages 2462-2476, XP007915048, ISSN: 1615-9853 [extrait le 2008-06-18]
- ANN-MARIE CALANDER ET AL: "Staphylococcus aureus infection triggers production of neutralizing,V8 protease-specic antibodies", FEMS IMMUNOLOGY AND MEDICAL MICROBIOLOGY, ELSEVIER SCIENCE B.V., AMSTERDAM, NL LNKD- DOI:10.1111/J.1574-695X.2007.00371.X, vol. 52, 1 janvier 2008 (2008-01-01), pages 267-272, XP007915049, ISSN: 0928-8244 [extrait le 2008-01-18]
- JACK GRUBER AND GEORGE G WRIGHT: "Ammonium Sulfate Coprecipitation Antibody Determination with Purified Staphylococcal Enterotoxins", JOURNAL OF BACTERIOLOGY, AMERICAN SOCIETY FOR MICROBIOLOGY, WASHINGTON, DC; US, vol. 99, no. 1, 1 juillet 1969 (1969-07-01), pages 18-24, XP007915050, ISSN: 0021-9193
- BERTIL CHRISTENSSON SVEN AKE HEDSTROM AND GORAN KRONVALL: "ANTIBODY RESPONSE TO ALPHA- AND BETAHEMOLYSIN FROM STAPHYLOCOCCUS A UREUS IN PATIENTS WITH STAPHYLOCOCCAL INFECTIONS AND IN NORMALS", ACTA PATHOLOGICA, MICROBIOLOGICA ET IMMUNOLOGICA SCANDINAVIC. SECTION B. MICROBIOLOGY,, vol. 91, 1 janvier 1983 (1983-01-01), pages 351-356, XP007915051, ISSN: 0180-0180
- STEPHEN ROYAN ET AL: "Identication of the secreted macromolecular immunogens of Staphylococcus aureus by analysis of serum", FEMS IMMUNOLOGY AND MEDICAL MICROBIOLOGY, ELSEVIER SCIENCE B.V., AMSTERDAM, NL, vol. 29, 1 janvier 2000 (2000-01-01), pages 315-321, XP007915052, ISSN: 0928-8244
- DRYLA AGNIESZKA ET AL: "Comparison of antibody repertoires against Staphylococcus aureus in healthy individuals and in acutely infected patients", CLINICAL AND DIAGNOSTIC LABORATORY IMMUNOLOGY, AMERICAN SOCIETY FOR MICROBIOLOGY, US LNKD- DOI:10.1128/CDLI.12.3.387-398.2005, vol. 12, no. 3, 1 mars 2005 (2005-03-01), pages 387-398, XP009094535, ISSN: 1071-412X
- VINH DONALD C ET AL: "Device-related infections: a review", JOURNAL OF LONG-TERM EFFECTS OF MEDICAL IMPLANTS, CRC PRESS, BOCA RATON, FL, US, vol. 15, no. 5, 1 janvier 2005 (2005-01-01), pages 467-488, XP009139088, ISSN: 1050-6934
- GEIPEL UDO: "Pathogenic organisms in hip joint infections", INTERNATIONAL JOURNAL OF MEDICAL SCIENCES, vol. 6, no. 5, 2 septembre 2009 (2009-09-02), pages 234-240, XP009139096, ISSN: 1449-1907 [extrait le 2009-09-02]
- CYNCYNATUS C ET AL: "K-08 Identification d'antigenes proteiques pour le serodiagnostic des infections sur protheses osteo-articulaires aStaphylococcus aureus", MEDECINE ET MALADIES INFECTIEUSES, SOCIETE FRANCAISE D'EDITIONS MEDICALES, PARIS, FR LNKD- DOI:10.1016/S0399-077X(09)74440-3, vol. 39, 19 juin 2009 (2009-06-19), page S54, XP026196660, ISSN: 0399-077X [extrait le 2009-06-01]
- HARRAGHY N ET AL: "The adhesive and immunomodulatong properties of the multifunctional staphylococcus aureus protein Eap", MICROBIOLOGY, SOCIETY FOR GENERAL MICROBIOLOGY, READING, GB, vol. 149, 1 janvier 2003 (2003-01-01), pages 2701-2707, XP002447471, ISSN: 1350-0872, DOI: DOI:10.1099/MIC.0.26465-0

### SEQUENCE LISTING

<110> InGen Biosciences
<120> Antigènes protéiques destinés au sérodiagnostic des infections à Staphylococcus aureus notamment sur prothèses ostéo-articulaires
<130> BET 10P0776
<150> US 61/213452
   <151> 2009-06-10
<160> 42
<170> PatentIn version 3.4
<210> 1
   <211> 308
   <212> PRT
   <213> Staphylococcus aureus
<400> 1
   Met Thr Asn Ser Ser Lys Ser Phe Thr Lys Phe Met Ala Ala Ser Ala
   1               5                   10                  15
   Val Phe Thr Met Gly Phe Leu Ser Val Pro Thr Ala Gly Ala Glu Gln
               20                  25                  30
   Thr Asn Gln Ile Ala Asn Lys Pro Gln Ala Ile Gln Trp His Thr Asn
           35                  40                  45
   Leu Thr Asn Glu Arg Phe Thr Thr Ile Ala His Arg Gly Ala Ser Gly
       50                  55                  60
   Tyr Ala Pro Glu His Thr Phe Gln Ala Tyr Asp Lys Ser His Asn Glu
   65                  70                  75                  80
   Leu Lys Ala Ser Tyr Ile Glu Ile Asp Leu Gln Arg Thr Lys Asp Gly
                   85                  90                  95
   His Leu Val Ala Met His Asp Glu Thr Val Asn Arg Thr Thr Asn Gly
               100                 105                 110
   His Gly Lys Val Glu Asp Tyr Thr Leu Asp Glu Leu Lys Gln Leu Asp
           115                 120                 125
   Ala Gly Ser Trp Phe Asn Lys Lys Tyr Pro Lys Tyr Ala Arg Ala Ser
       130                 135                 140
   Tyr Lys Asn Ala Lys Val Pro Thr Leu Asp Glu Ile Leu Glu Arg Tyr
   145                 150                 155                 160
   Gly Pro Asn Ala Asn Tyr Tyr Ile Glu Thr Lys Ser Pro Asp Val Tyr
                   165                 170                 175
   Pro Gly Met Glu Glu Gln Leu Leu Ala Ser Leu Lys Lys His His Leu
   180 185 190
   Leu Asn Asn Asn Lys Leu Lys Asn Gly His Val Met Ile Gln Ser Phe
   195 200 205
   Ser Asp Glu Ser Leu Lys Lys Ile His Arg Gln Asn Lys His Val Pro
   210 215 220
   Leu Val Lys Val Asp Lys Gly Glu Leu Gln Gln Phe Asn Asp Gln Arg
   225 230 235 240
   Leu Lys Glu Ile Arg Ser Tyr Ala Ile Gly Leu Gly Pro Asp Tyr Thr
   245 250 255
   Asp Leu Thr Glu Gln Asn Thr His His Leu Lys Asp Leu Gly Phe Ile
   260 265 270
   Val His Pro Tyr Thr Val Asn Glu Lys Ala Asp Met Leu Arg Leu Asn
   275 280 285
   Lys Tyr Gly Val Asp Gly Val Phe Thr Asn Phe Ala Asp Lys Tyr Lys
   290 295 300
   Glu Val Ile Lys
   305
<210> 2
   <211> 1248
   <212> PRT
   <213> Staphylococcus aureus
<400> 2
   Met Ala Lys Lys Phe Asn Tyr Lys Leu Pro Ser Met Val Ala Leu Thr
   1 5 10 15
   Leu Val Gly Ser Ala Val Thr Ala His Gln Val Gln Ala Ala Glu Thr
   20 25 30
   Thr Gln Asp Gln Thr Thr Asn Lys Asn Val Leu Asp Ser Asn Lys Val
   35 40 45
   Lys Ala Thr Thr Glu Gln Ala Lys Ala Glu Val Lys Asn Pro Thr Gln
   50 55 60
   Asn Ile Ser Gly Thr Gln Val Tyr Gln Asp Pro Ala Ile Val Gln Pro
   65 70 75 80
   Lys Thr Ala Asn Asn Lys Thr Gly Asn Ala Gln Val Ser Gln Lys Val
                   85                  90                  95
   Asp Thr Ala Gln Val Asn Gly Asp Thr Arg Ala Asn Gln Ser Ala Thr
               100                 105                 110
   Thr Asn Asn Thr Gln Pro Val Ala Lys Ser Thr Ser Thr Thr Ala Pro
           115                 120                 125
   Lys Thr Asn Thr Asn Val Thr Asn Ala Gly Tyr Ser Leu Val Asp Asp
       130                 135                 140
   Glu Asp Asp Asn Ser Glu His Gln Ile Asn Pro Glu Leu Ile Lys Ser
   145                 150                 155                 160
   Ala Ala Lys Pro Ala Ala Leu Glu Thr Gln Tyr Lys Ala Ala Ala Pro
                   165                 170                 175
   Lys Ala Lys Thr Glu Ala Thr Pro Lys Val Thr Thr Phe Ser Ala Ser
               180                 185                 190
   Ala Gln Pro Arg Ser Val Ala Ala Thr Pro Lys Thr Ser Leu Pro Lys
           195                 200                 205
   Tyr Lys Pro Gln Val Asn Ser Ser Ile Asn Asp Tyr Ile Arg Lys Asn
       210                 215                 220
   Asn Leu Lys Ala Pro Lys Ile Glu Glu Asp Tyr Thr Ser Tyr Phe Pro
   225                 230                 235                 240
   Lys Tyr Ala Tyr Arg Asn Gly Val Gly Arg Pro Glu Gly Ile Val Val
                   245                 250                 255
   His Asp Thr Ala Asn Asp Arg Ser Thr Ile Asn Gly Glu Ile Ser Tyr
               260                 265                 270
   Met Lys Asn Asn Tyr Gln Asn Ala Phe Val His Ala Phe Val Asp Gly
           275                 280                 285
   Asp Arg Ile Ile Glu Thr Ala Pro Thr Asp Tyr Leu Ser Trp Gly Val
       290                 295                 300
   Gly Ala Val Gly Asn Pro Arg Phe Ile Asn Val Glu Ile Val His Thr
   305                 310                 315                 320
   His Asp Tyr Ala Ser Phe Ala Arg Ser Met Asn Asn Tyr Ala Asp Tyr
                   325                 330                 335
   Ala Ala Thr Gln Leu Gln Tyr Tyr Gly Leu Lys Pro Asp Ser Ala Glu
               340                 345                 350
   Tyr Asp Gly Asn Gly Thr Val Trp Thr His Tyr Ala Val Ser Lys Tyr
           355                 360                 365
   Leu Gly Gly Thr Asp His Ala Asp Pro His Gly Tyr Leu Arg Ser His
       370                 375                 380
   Asn Tyr Ser Tyr Asp Gln Leu Tyr Asp Leu Ile Asn Glu Lys Tyr Leu
   385                 390                 395                 400
   Ile Lys Met Gly Lys Val Ala Pro Trp Gly Thr Gln Phe Thr Thr Thr
                   405                 410                 415
   Pro Thr Thr Pro Ser Lys Pro Thr Thr Pro Ser Lys Pro Ser Thr Gly
               420                 425                 430
   Lys Leu Thr Val Ala Ala Asn Asn Gly Val Ala Gln Ile Lys Pro Thr
           435                 440                 445
   Asn Ser Gly Leu Tyr Thr Thr Val Tyr Asp Lys Thr Gly Lys Ala Thr
       450                 455                 460
   Asn Glu Val Gln Lys Thr Phe Ala Val Ser Lys Thr Ala Thr Leu Gly
   465                 470                 475                 480
   Asn Gln Lys Phe Tyr Leu Val Gln Asp Tyr Asn Ser Gly Asn Lys Phe
                   485                 490                 495
   Gly Trp Val Lys Glu Gly Asp Val Val Tyr Asn Thr Ala Lys Ser Pro
               500                 505                 510
   Val Asn Val Asn Gln Ser Tyr Ser Ile Lys Ser Gly Thr Lys Leu Tyr
           515                 520                 525
   Thr Val Pro Trp Gly Thr Ser Lys Gln Val Ala Gly Ser Val Ser Gly
       530                 535                 540
   Ser Gly Asn Gln Thr Phe Lys Ala Ser Lys Gln Gln Gln Ile Asp Lys
   545                 550                 555                 560
   Ser Ile Tyr Leu Tyr Gly Ser Val Asn Gly Lys Ser Gly Trp Val Ser
                   565                 570                 575
   Lys Ala Tyr Leu Val Asp Thr Ala Lys Pro Thr Pro Thr Pro Ile Pro
               580                 585                 590
   Lys Pro Ser Thr Pro Thr Thr Asn Asn Lys Leu Thr Val Ser Ser Leu
           595                 600                 605
   Asn Gly Val Ala Gln Ile Asn Ala Lys Asn Asn Gly Leu Phe Thr Thr
   610 615 620
   Val Tyr Asp Lys Thr Gly Lys Pro Thr Lys Glu Val Gln Lys Thr Phe
   625 630 635 640
   Ala Val Thr Lys Glu Ala Ser Leu Gly Gly Asn Lys Phe Tyr Leu Val
   645 650 655
   Lys Asp Tyr Asn Ser Pro Thr Leu Ile Gly Trp Val Lys Gln Gly Asp
   660 665 670
   Val Ile Tyr Asn Asn Ala Lys Ser Pro Val Asn Val Met Gln Thr Tyr
   675 680 685
   Thr Val Lys Pro Gly Thr Lys Leu Tyr Ser Val Pro Trp Gly Thr Tyr
   690 695 700
   Lys Gln Glu Ala Gly Ala Val Ser Gly Thr Gly Asn Gln Thr Phe Lys
   705 710 715 720
   Ala Thr Lys Gln Gln Gln Ile Asp Lys Ser Ile Tyr Leu Phe Gly Thr
   725 730 735
   Val Asn Gly Lys Ser Gly Trp Val Ser Lys Ala Tyr Leu Ala Val Pro
   740 745 750
   Ala Ala Pro Lys Lys Ala Val Ala Gln Pro Lys Thr Ala Val Lys Ala
   755 760 765
   Tyr Thr Val Thr Lys Pro Gln Thr Thr Gln Thr Val Ser Lys Ile Ala
   770 775 780
   Gln Val Lys Pro Asn Asn Thr Gly Ile Arg Ala Ser Val Tyr Glu Lys
   785 790 795 800
   Thr Ala Lys Asn Gly Ala Lys Tyr Ala Asp Arg Thr Phe Tyr Val Thr
   805 810 815
   Lys Glu Arg Ala His Gly Asn Glu Thr Tyr Val Leu Leu Asn Asn Thr
   820 825 830
   Ser His Asn Ile Pro Leu Gly Trp Phe Asn Val Lys Asp Leu Asn Val
   835 840 845
   Gln Asn Leu Gly Lys Glu Val Lys Thr Thr Gln Lys Tyr Thr Val Asn
   850 855 860
   Lys Ser Asn Asn Gly Leu Ser Met Val Pro Trp Gly Thr Lys Asn Gln
   865 870 875 880
   Val Ile Leu Thr Gly Asn Asn Ile Ala Gln Gly Thr Phe Asn Ala Thr
   885 890 895
   Lys Gln Val Ser Val Gly Lys Asp Val Tyr Leu Tyr Gly Thr Ile Asn
   900 905 910
   Asn Arg Thr Gly Trp Val Asn Ala Lys Asp Leu Thr Ala Pro Thr Ala
   915 920 925
   Val Lys Pro Thr Thr Ser Ala Ala Lys Asp Tyr Asn Tyr Thr Tyr Val
   930 935 940
   Ile Lys Asn Gly Asn Gly Tyr Tyr Tyr Val Thr Pro Asn Ser Asp Thr
   945 950 955 960
   Ala Lys Tyr Ser Leu Lys Ala Phe Asn Glu Gln Pro Phe Ala Val Val
   965 970 975
   Lys Glu Gln Val Ile Asn Gly Gln Thr Trp Tyr Tyr Gly Lys Leu Ser
   980 985 990
   Asn Gly Lys Leu Ala Trp Ile Lys Ser Thr Asp Leu Ala Lys Glu Leu
   995 1000 1005
   Ile Lys Tyr Asn Gln Thr Gly Met Thr Leu Asn Gln Val Ala Gln
   1010 1015 1020
   Ile Gln Ala Gly Leu Gln Tyr Lys Pro Gln Val Gln Arg Val Pro
   1025 1030 1035
   Gly Lys Trp Thr Asp Ala Asn Phe Asn Asp Val Lys His Ala Met
   1040 1045 1050
   Asp Thr Lys Arg Leu Ala Gln Asp Pro Ala Leu Lys Tyr Gln Phe
   1055 1060 1065
   Leu Arg Leu Asp Gln Pro Gln Asn Ile Ser Ile Asp Lys Ile Asn
   1070 1075 1080
   Gln Phe Leu Lys Gly Lys Gly Val Leu Glu Asn Gln Gly Ala Ala
   1085 1090 1095
   Phe Asn Lys Ala Ala Gln Met Tyr Gly Ile Asn Glu Val Tyr Leu
   1100 1105 1110
   Ile Ser His Ala Leu Leu Glu Thr Gly Asn Gly Thr Ser Gln Leu
       1115                1120                1125
   Ala Lys Gly Ala Asp Val Val Asn Asn Lys Val Val Thr Asn Ser
       1130                1135                1140
   Asn Thr Lys Tyr His Asn Val Phe Gly Ile Ala Ala Tyr Asp Asn
       1145                1150                1155
   Asp Pro Leu Arg Glu Gly Ile Lys Tyr Ala Lys Gln Ala Gly Trp
       1160                1165                1170
   Asp Thr Val Ser Lys Ala Ile Val Gly Gly Ala Lys Phe Ile Gly
       1175                1180                1185
   Asn Ser Tyr Val Lys Ala Gly Gln Asn Thr Leu Tyr Lys Met Arg
       1190                1195                1200
   Trp Asn Pro Ala His Pro Gly Thr His Gln Tyr Ala Thr Asp Val
       1205                1210                1215
   Asp Trp Ala Asn Ile Asn Ala Lys Ile Ile Lys Gly Tyr Tyr Asp
       1220                1225                1230
   Lys Ile Gly Glu Val Gly Lys Tyr Phe Asp Ile Pro Gln Tyr Lys
       1235                1240                1245
<210> 3
   <211> 691
   <212> PRT
   <213> Staphylococcus aureus
<400> 3
   Met Leu Arg Gly Gln Glu Glu Arg Lys Tyr Ser Ile Arg Lys Tyr Ser
   1               5                   10                  15
   Ile Gly Val Val Ser Val Leu Ala Ala Thr Met Phe Val Val Ser Ser
               20                  25                  30
   His Glu Ala Gln Ala Ser Glu Lys Thr Pro Thr Ser Asn Ala Ala Ala
           35                  40                  45
   Gln Lys Glu Thr Leu Asn Gln Pro Gly Glu Gln Gly Asn Ala Ile Thr
       50                  55                  60
   Ser His Gln Met Gln Ser Gly Lys Gln Leu Asp Asp Met His Lys Glu
   65                  70                  75                  80
   Asn Gly Lys Ser Gly Thr Val Thr Glu Gly Lys Asp Thr Leu Gln Ser
                   85                  90                  95
   Ser Lys His Gln Ser Thr Gln Asn Ser Lys Thr Ile Arg Thr Gln Asn
   100 105 110
   Asp Asn Gln Val Lys Gln Asp Ser Glu Arg Gln Gly Ser Lys Gln Ser
   115 120 125
   His Gln Asn Asn Ala Thr Asn Asn Thr Glu Arg Gln Asn Asp Gln Val
   130 135 140
   Gln Asn Thr His His Ala Glu Arg Asn Gly Ser Gln Ser Thr Thr Ser
   145 150 155 160
   Gln Ser Asn Asp Val Asp Lys Ser Gln Pro Ser Ile Pro Ala Gln Lys
   165 170 175
   Val Leu Pro Asn His Asp Lys Ala Ala Pro Thr Ser Thr Thr Pro Pro
   180 185 190
   Ser Asn Asp Lys Thr Ala Pro Lys Ser Thr Lys Ala Gln Asp Ala Thr
   195 200 205
   Thr Asp Lys His Pro Asn Gln Gln Asp Thr His Gln Pro Ala His Gln
   210 215 220
   Ile Ile Asp Ala Lys Gln Asp Asp Thr Val Arg Gln Ser Glu Gln Lys
   225 230 235 240
   Pro Gln Val Gly Asp Leu Ser Lys His Ile Asp Gly Gln Asn Ser Pro
   245 250 255
   Glu Lys Pro Thr Asp Lys Asn Thr Asp Asn Lys Gln Leu Ile Lys Asp
   260 265 270
   Ala Leu Gln Ala Pro Lys Thr Arg Ser Thr Thr Asn Ala Ala Ala Asp
   275 280 285
   Ala Lys Lys Val Arg Pro Leu Lys Ala Asn Gln Val Gln Pro Leu Asn
   290 295 300
   Lys Tyr Pro Val Val Phe Val His Gly Phe Leu Gly Leu Val Gly Asp
   305 310 315 320
   Asn Ala Pro Ala Leu Tyr Pro Asn Tyr Trp Gly Gly Asn Lys Phe Lys
   325 330 335
   Val Ile Glu Glu Leu Arg Lys Gln Gly Tyr Asn Val His Gln Ala Ser
   340 345 350
   Val Ser Ala Phe Gly Ser Asn Tyr Asp Arg Ala Val Glu Leu Tyr Tyr
           355                 360                 365
   Tyr Ile Lys Gly Gly Arg Val Asp Tyr Gly Ala Ala His Ala Ala Lys
       370                 375                 380
   Tyr Gly His Glu Arg Tyr Gly Lys Thr Tyr Lys Gly Ile Met Pro Asn
   385                 390                 395                 400
   Trp Glu Pro Gly Lys Lys Val His Leu Val Gly His Ser Met Gly Gly
                   405                 410                 415
   Gln Thr Ile Arg Leu Met Glu Glu Phe Leu Arg Asn Gly Asn Lys Glu
               420                 425                 430
   Glu Ile Ala Tyr His Lys Ala His Gly Gly Glu Ile Ser Pro Leu Phe
           435                 440                 445
   Thr Gly Gly His Asn Asn Met Val Ala Ser Ile Thr Thr Leu Ala Thr
       450                 455                 460
   Pro His Asn Gly Ser Gln Ala Ala Asp Lys Phe Gly Asn Thr Glu Ala
   465                 470                 475                 480
   Val Arg Lys Ile Met Phe Ala Leu Asn Arg Phe Met Gly Asn Lys Tyr
                   485                 490                 495
   Ser Asn Ile Asp Leu Gly Leu Thr Gln Trp Gly Phe Lys Gln Leu Pro
               500                 505                 510
   Asn Glu Ser Tyr Ile Asp Tyr Ile Lys Arg Val Ser Lys Ser Lys Ile
           515                 520                 525
   Trp Thr Ser Asp Asp Asn Ala Ala Tyr Asp Leu Thr Leu Asp Gly Ser
       530                 535                 540
   Ala Lys Leu Asn Asn Met Thr Ser Met Asn Pro Asn Ile Thr Tyr Thr
   545                 550                 555                 560
   Thr Tyr Thr Gly Val Ser Ser His Thr Gly Pro Leu Gly Tyr Glu Asn
                   565                 570                 575
   Pro Asp Leu Gly Thr Phe Phe Leu Met Asp Thr Thr Ser Arg Ile Ile
               580                 585                 590
   Gly His Asp Ala Arg Glu Glu Trp Arg Lys Asn Asp Gly Val Val Pro
           595                 600                 605
   Val Ile Ser Ser Leu His Pro Ser Asn Gln Pro Phe Ile Asn Val Thr
       610                 615                 620
   Asn Asp Glu Pro Ala Thr Arg Arg Gly Ile Trp Gln Val Lys Pro Ile
   625                 630                 635                 640
   Ile Gln Gly Trp Asp His Val Asp Phe Ile Gly Val Asp Phe Leu Asp
                   645                 650                 655
   Phe Lys Arg Lys Gly Ala Glu Leu Ala Asn Phe Tyr Thr Gly Ile Ile
               660                 665                 670
   Asn Asp Leu Leu Arg Val Glu Ala Thr Glu Ser Lys Gly Thr Gln Leu
           675                 680                 685
   Lys Ala Ser
       690
<210> 4
   <211> 658
   <212> PRT
   <213> Staphylococcus aureus
<400> 4
   Met Lys Lys Gln Ile Ile Ser Leu Gly Ala Leu Ala Val Ala Ser Ser
   1               5                   10                  15
   Leu Phe Thr Trp Asp Asn Lys Ala Asp Ala Ile Val Thr Lys Asp Tyr
               20                  25                  30
   Ser Lys Glu Ser Arg Val Asn Glu Lys Ser Lys Lys Gly Ala Thr Val
           35                  40                  45
   Ser Asp Tyr Tyr Tyr Trp Lys Ile Ile Asp Ser Leu Glu Ala Gln Phe
       50                  55                  60
   Thr Gly Ala Ile Asp Leu Leu Glu Asp Tyr Lys Tyr Gly Asp Pro Ile
   65                  70                  75                  80
   Tyr Lys Glu Ala Lys Asp Arg Leu Met Thr Arg Val Leu Gly Glu Asp
                   85                  90                  95
   Gln Tyr Leu Leu Lys Lys Lys Ile Asp Glu Tyr Glu Leu Tyr Lys Lys
               100                 105                 110
   Trp Tyr Lys Ser Ser Asn Lys Asn Thr Asn Met Leu Thr Phe His Lys
           115                 120                 125
   Tyr Asn Leu Tyr Asn Leu Thr Met Asn Glu Tyr Asn Asp Ile Phe Asn
       130                 135                 140
   Ser Leu Lys Asp Ala Val Tyr Gln Phe Asn Lys Glu Val Lys Glu Ile
   145                 150                 155                 160
   Glu His Lys Asn Val Asp Leu Lys Gln Phe Asp Lys Asp Gly Glu Asp
                   165                 170                 175
   Lys Ala Thr Lys Glu Val Tyr Asp Leu Val Ser Glu Ile Asp Thr Leu
               180                 185                 190
   Val Val Thr Tyr Tyr Ala Asp Lys Asp Tyr Gly Glu His Ala Lys Glu
           195                 200                 205
   Leu Arg Ala Lys Leu Asp Leu Ile Leu Gly Asp Thr Asp Asn Pro His
       210                 215                 220
   Lys Ile Thr Asn Glu Arg Ile Lys Lys Glu Met Ile Asp Asp Leu Asn
   225                 230                 235                 240
   Ser Ile Ile Asp Asp Phe Phe Met Glu Thr Lys Gln Asn Arg Pro Asn
                   245                 250                 255
   Ser Ile Thr Lys Tyr Asp Pro Thr Lys His Asn Phe Lys Glu Lys Ser
               260                 265                 270
   Glu Asn Lys Pro Asn Phe Asp Lys Leu Val Glu Glu Thr Lys Lys Ala
           275                 280                 285
   Val Lys Glu Ala Asp Glu Ser Trp Lys Asn Lys Thr Val Lys Lys Tyr
       290                 295                 300
   Glu Glu Thr Val Thr Lys Ser Pro Val Val Lys Glu Glu Lys Lys Val
   305                 310                 315                 320
   Glu Glu Pro Gln Leu Pro Lys Val Gly Asn Gln Gln Glu Val Lys Thr
                   325                 330                 335
   Thr Ala Gly Lys Ala Glu Glu Thr Thr Gln Pro Val Ala Gln Pro Leu
               340                 345                 350
   Val Lys Ile Pro Gln Glu Thr Ile Tyr Gly Glu Thr Val Lys Gly Pro
           355                 360                 365
   Glu Tyr Pro Thr Met Glu Asn Lys Thr Leu Gln Gly Glu Ile Val Gln
       370                 375                 380
   Gly Pro Asp Phe Leu Thr Met Glu Gln Asn Arg Pro Ser Leu Ser Asp
   385                 390                 395                 400
   Asn Tyr Thr Gln Pro Thr Thr Pro Asn Pro Ile Leu Glu Gly Leu Glu
   405 410 415
   Gly Ser Ser Ser Lys Leu Glu Ile Lys Pro Gln Gly Thr Glu Ser Thr
   420 425 430
   Leu Lys Gly Ile Gln Gly Glu Ser Ser Asp Ile Glu Val Lys Pro Gln
   435 440 445
   Ala Thr Glu Thr Thr Glu Ala Ser Gln Tyr Gly Pro Arg Pro Gln Phe
   450 455 460
   Asn Lys Thr Pro Lys Tyr Val Lys Tyr Arg Asp Ala Gly Thr Gly Ile
   465 470 475 480
   Arg Glu Tyr Asn Asp Gly Thr Phe Gly Tyr Glu Ala Arg Pro Arg Phe
   485 490 495
   Asn Lys Pro Ser Glu Thr Asn Ala Tyr Asn Val Thr Thr Asn Gln Asp
   500 505 510
   Gly Thr Val Ser Tyr Gly Ala Arg Pro Thr Gln Asn Lys Pro Ser Glu
   515 520 525
   Thr Asn Ala Tyr Asn Val Thr Thr His Ala Asn Gly Gln Val Ser Tyr
   530 535 540
   Gly Ala Arg Pro Thr Gln Lys Lys Pro Ser Lys Thr Asn Ala Tyr Asn
   545 550 555 560
   Val Thr Thr His Ala Asn Gly Gln Val Ser Tyr Gly Ala Arg Pro Thr
   565 570 575
   Gln Lys Lys Pro Ser Lys Thr Asn Ala Tyr Asn Val Thr Thr His Ala
   580 585 590
   Asn Gly Gln Val Ser Tyr Gly Ala Arg Pro Thr Tyr Lys Lys Pro Ser
   595 600 605
   Glu Thr Asn Ala Tyr Asn Val Thr Thr His Ala Asn Gly Gln Val Ser
   610 615 620
   Tyr Gly Ala Arg Pro Thr Gln Lys Lys Pro Ser Glu Thr Asn Ala Tyr
   625 630 635 640
   Asn Val Thr Thr His Ala Asp Gly Thr Ala Thr Tyr Gly Pro Arg Val
   645 650 655
   Thr Lys
<210> 5
   <211> 488
   <212> PRT
   <213> Staphylococcus aureus
<400> 5
   Met Trp Glu Ser Lys Phe Ala Lys Glu Ser Leu Thr Phe Asp Asp Val
   1               5                   10                  15
   Leu Leu Ile Pro Ala Gln Ser Asp Ile Leu Pro Lys Asp Val Asp Leu
               20                  25                  30
   Ser Val Gln Leu Ser Asp Lys Val Lys Leu Asn Ile Pro Val Ile Ser
           35                  40                  45
   Ala Gly Met Asp Thr Val Thr Glu Ser Lys Met Ala Ile Ala Met Ala
       50                  55                  60
   Arg Gln Gly Gly Leu Gly Val Ile His Lys Asn Met Gly Val Glu Glu
   65                  70                  75                  80
   Gln Ala Asp Glu Val Gln Lys Val Lys Arg Ser Glu Asn Gly Val Ile
                   85                  90                  95
   Ser Asn Pro Phe Phe Leu Thr Pro Glu Glu Ser Val Tyr Glu Ala Glu
               100                 105                 110
   Ala Leu Met Gly Lys Tyr Arg Ile Ser Gly Val Pro Ile Val Asp Asn
           115                 120                 125
   Lys Glu Asp Arg Asn Leu Val Gly Ile Leu Thr Asn Arg Asp Leu Arg
       130                 135                 140
   Phe Ile Glu Asp Phe Ser Ile Lys Ile Val Asp Val Met Thr Gln Glu
   145                 150                 155                 160
   Asn Leu Ile Thr Ala Pro Val Asn Thr Thr Leu Glu Glu Ala Glu Lys
                   165                 170                 175
   Ile Leu Gln Lys His Lys Ile Glu Lys Leu Pro Leu Val Lys Asp Gly
               180                 185                 190
   Arg Leu Glu Gly Leu Ile Thr Ile Lys Asp Ile Glu Lys Val Ile Glu
           195                 200                 205
   Phe Pro Asn Ala Ala Lys Asp Glu His Gly Arg Leu Leu Val Ala Ala
       210                 215                 220
   Ala Ile Gly Ile Ser Lys Asp Thr Asp Ile Arg Ala Gln Lys Leu Val
   225                 230                 235                 240
   Glu Ala Gly Val Asp Val Leu Val Ile Asp Thr Ala His Gly His Ser
                   245                 250                 255
   Lys Gly Val Ile Asp Gln Val Lys His Ile Lys Lys Thr Tyr Pro Glu
               260                 265                 270
   Ile Thr Leu Val Ala Gly Asn Val Ala Thr Ala Glu Ala Thr Lys Asp
           275                 280                 285
   Leu Phe Glu Ala Gly Ala Asp Ile Val Lys Val Gly Ile Gly Pro Gly
       290                 295                 300
   Ser Ile Cys Thr Thr Arg Val Val Ala Gly Val Gly Val Pro Gln Ile
   305                 310                 315                 320
   Thr Ala Ile Tyr Asp Cys Ala Thr Glu Ala Arg Lys His Gly Lys Ala
                   325                 330                 335
   Ile Ile Ala Asp Gly Gly Ile Lys Phe Ser Gly Asp Ile Ile Lys Ala
               340                 345                 350
   Leu Ala Ala Gly Gly His Ala Val Met Leu Gly Ser Leu Leu Ala Gly
           355                 360                 365
   Thr Glu Glu Ser Pro Gly Ala Thr Glu Ile Phe Gln Gly Arg Gln Tyr
       370                 375                 380
   Lys Val Tyr Arg Gly Met Gly Ser Leu Gly Ala Met Glu Lys Gly Ser
   385                 390                 395                 400
   Asn Asp Arg Tyr Phe Gln Glu Asp Lys Ala Pro Lys Lys Phe Val Pro
                   405                 410                 415
   Glu Gly Ile Glu Gly Arg Thr Ala Tyr Lys Gly Ala Leu Gln Asp Thr
               420                 425                 430
   Ile Tyr Gln Leu Met Gly Gly Val Arg Ala Gly Met Gly Tyr Thr Gly
           435                 440                 445
   Ser His Asp Leu Arg Glu Leu Arg Glu Glu Ala Gln Phe Thr Arg Met
       450                 455                 460
   Gly Pro Ala Gly Leu Ala Glu Ser His Pro His Asn Ile Gln Ile Thr
   465                 470                 475                 480
   Lys Glu Ser Pro Asn Tyr Ser Phe
   485
<210> 6
   <211> 495
   <212> PRT
   <213> Staphylococcus aureus
<400> 6
   Met Arg Glu Asn Phe Lys Leu Arg Lys Met Lys Val Gly Leu Val Ser
   1               5                   10                  15
   Val Ala Ile Thr Met Leu Tyr Ile Met Thr Asn Gly Gln Ala Glu Ala
               20                  25                  30
   Ser Glu Thr Asn Gln Lys Val Ser Thr Asn Gln Glu Ser Lys Ala Val
           35                  40                  45
   Ser Gln Thr Glu Gln Asn Ser Lys Glu Thr Lys Ala Ala Glu Ser Ser
       50                  55                  60
   Lys Asn Phe Val Asn Leu Asp Pro Ile Lys Pro Gly Ala Gln Lys Val
   65                  70                  75                  80
   Thr Gly Thr Thr Leu Pro Asn His Ile Ile Leu Leu Asn Ile Asp Gly
                   85                  90                  95
   Lys Ser Ala Asp Ser Val Glu Gly Gly Tyr Gly Asp Phe Ile Thr Ala
               100                 105                 110
   Asn Asp Lys Gly Glu Phe Glu Tyr Pro Leu Asn Asn Arg Lys Ile Val
           115                 120                 125
   His Asn Gln Glu Ile Glu Val Ser Ser Ser Ser Pro Asp Leu Gly Asp
       130                 135                 140
   Asp Glu Glu Asp Glu Glu Val Glu Glu Ser Ser Thr Glu Lys Ala Gly
   145                 150                 155                 160
   Ala Glu Glu Glu Ser Thr Asp Ala Lys Ala Thr Tyr Thr Thr Pro Arg
                   165                 170                 175
   Tyr Glu Lys Ala Tyr Glu Ile Pro Lys Glu Gln Leu Lys Glu Lys Asp
               180                 185                 190
   Gly His His Gln Val Phe Ile Glu Pro Ile Thr Glu Gly Ser Gly Ile
           195                 200                 205
   Ile Lys Gly His Thr Ser Val Lys Gly Lys Val Ala Leu Ser Ile Asn
   210 215 220
   Asn Lys Phe Ile Asn Phe Glu Glu Arg Ala Lys Gly Gly Ile Ser Lys
   225 230 235 240
   Glu Asp Thr Lys Ala Ser Ser Asp Gly Val Trp Met Pro Ile Asn Glu
   245 250 255
   Lys Gly Tyr Phe Asp Phe Asp Phe Lys Lys Asn Pro Phe Asp Asn Leu
   260 265 270
   Glu Leu Lys Lys Asn Asp Glu Ile Ser Leu Thr Phe Ala Pro Asp Asp
   275 280 285
   Glu Asp Glu Ala Leu Lys Ser Leu Ile Phe Lys Thr Lys Val Thr Ser
   290 295 300
   Leu Glu Asp Ile Asp Lys Ala Glu Thr Lys Tyr Asp His Thr Lys Val
   305 310 315 320
   Glu Lys Val Lys Val Leu Lys Asp Val Lys Glu Asp Ile His Val Asp
   325 330 335
   Glu Ile Tyr Gly Ser Leu Tyr His Thr Glu Lys Gly Lys Gly Ile Leu
   340 345 350
   Asp Lys Gln Gly Thr Lys Glu Ile Thr Gly Lys Thr Lys Phe Ala Asn
   355 360 365
   Ala Val Val Lys Val Tyr Ser Asp Leu Gly Asp Ala Gln Leu Phe Pro
   370 375 380
   Asp Ile Gln Val Asp Glu Asn Gly Lys Phe Ser Phe Asp Ala Glu Lys
   385 390 395 400
   Ala Gly Phe Arg Leu Gln Asn Gly Glu Thr Leu Asn Phe Ala Val Val
   405 410 415
   Lys Pro Ile Thr Gly Asp Leu Leu His Gln Gly Phe Val Ser Lys Tyr
   420 425 430
   Ile Asp Val Tyr Glu Ser Pro Glu Glu Lys Lys Glu Arg Glu Phe Glu
   435 440 445
   Glu Lys Leu Glu Asn Thr Pro Ala Tyr His Lys Leu His Gly Asp Lys
   450 455 460
   Ile Val Gly Tyr Asp Val Gln Gly Asn Pro Ser Thr Trp Phe Tyr Pro
   465 470 475 480
   Leu Gly Glu Lys Lys Val Glu Arg Thr Thr Pro Lys Leu Glu Lys
   485 490 495
<210> 7
   <211> 468
   <212> PRT
   <213> Staphylococcus aureus
<400> 7
   Met Thr Gln Gln Ile Gly Val Ile Gly Leu Ala Val Met Gly Lys Asn
   1 5 10 15
   Leu Ala Trp Asn Ile Glu Ser His Gly Tyr Ser Val Ser Val Phe Asn
   20 25 30
   Arg Ser Ser Glu Lys Thr Asp Leu Met Val Glu Glu Ser Lys Gly Lys
   35 40 45
   Asn Ile His Pro Thr Tyr Ser Leu Glu Glu Phe Val Asn Ser Leu Glu
   50 55 60
   Lys Pro Arg Lys Ile Leu Leu Met Val Gln Ala Gly Lys Ala Thr Asp
   65 70 75 80
   Ala Thr Ile Asp Ser Leu Leu Pro Leu Leu Asp Asp Gly Asp Ile Leu
   85 90 95
   Ile Asp Gly Gly Asn Thr Asn Tyr Gln Asp Thr Ile Arg Arg Asn Lys
   100 105 110
   Ala Leu Ala Gln Ser Ala Ile Asn Phe Ile Gly Met Gly Val Ser Gly
   115 120 125
   Gly Glu Ile Gly Ala Leu Thr Gly Pro Ser Leu Met Pro Gly Gly Gln
   130 135 140
   Glu Glu Ala Tyr Asn Lys Val Ala Asp Ile Leu Asp Ala Ile Ala Ala
   145 150 155 160
   Lys Ala Lys Asp Gly Ala Ser Cys Val Thr Tyr Ile Gly Pro Asn Gly
   165 170 175
   Ala Gly His Tyr Val Lys Met Val His Asn Gly Ile Glu Tyr Ala Asp
   180 185 190
   Met Gln Leu Ile Ala Glu Ser Tyr Ala Met Met Lys Glu Leu Leu Gly
   195 200 205
   Met Ser His Glu Asp Ile Ala Gln Thr Phe Lys Asp Trp Asn Ala Gly
   210 215 220
   Glu Leu Glu Ser Tyr Leu Ile Glu Ile Thr Gly Asp Ile Phe Met Lys
   225 230 235 240
   Leu Asp Glu Asn Lys Glu Ala Leu Val Glu Lys Ile Leu Asp Thr Ala
   245 250 255
   Gly Gln Lys Gly Thr Gly Lys Trp Thr Ser Ile Asn Ala Leu Glu Leu
   260 265 270
   Gly Ile Pro Leu Thr Ile Ile Thr Glu Ser Val Phe Ala Arg Phe Ile
   275 280 285
   Ser Ser Ile Lys Glu Glu Arg Val Asn Ala Ser Lys Glu Leu Asn Gly
   290 295 300
   Pro Lys Ala Ser Phe Asp Gly Asp Lys Lys Asp Phe Leu Glu Lys Ile
   305 310 315 320
   Arg Lys Ala Leu Tyr Met Ser Lys Ile Cys Ser Tyr Ala Gln Gly Phe
   325 330 335
   Ala Gln Met Arg Lys Ala Ser Glu Asp Asn Glu Trp Asn Leu Lys Leu
   340 345 350
   Gly Asp Leu Ala Met Ile Trp Arg Glu Gly Cys Ile Ile Arg Ala Gln
   355 360 365
   Phe Leu Gln Lys Ile Lys Asp Ala Tyr Asp Asn Asn Pro Gly Leu Gln
   370 375 380
   Asn Leu Leu Leu Asp Pro Tyr Phe Lys Asn Ile Val Thr Glu Tyr Gln
   385 390 395 400
   Asp Ala Leu Arg Asp Val Val Ala Thr Gly Val Gln Asn Gly Val Pro
   405 410 415
   Thr Pro Gly Phe Ser Ser Ser Ile Asn Tyr Tyr Asp Ser Tyr Arg Ala
   420 425 430
   Ala Asp Leu Pro Ala Asn Leu Ile Gln Ala Gln Arg Asp Tyr Phe Gly
   435 440 445
   Ala His Thr Tyr Glu Arg Lys Asp Lys Glu Gly Val Phe His Thr Gln
   450 455 460
   Trp Ile Glu Glu
   465
<210> 8
   <211> 450
   <212> PRT
   <213> Staphylococcus aureus
<400> 8
   Met Lys Lys Lys Asn Ile Tyr Ser Ile Arg Lys Leu Gly Val Gly Ile
   1               5                   10                  15
   Ala Ser Val Thr Leu Gly Thr Leu Leu Ile Ser Gly Gly Val Thr Pro
               20                  25                  30
   Ala Ala Asn Ala Ala Gln His Asp Glu Ala Gln Gln Asn Ala Phe Tyr
           35                  40                  45
   Gln Val Leu Asn Met Pro Asn Leu Asn Ala Asp Gln Arg Asn Gly Phe
       50                  55                  60
   Ile Gln Ser Leu Lys Asp Asp Pro Ser Gln Ser Ala Asn Val Leu Gly
   65                  70                  75                  80
   Glu Ala Gln Lys Leu Asn Asp Ser Gln Ala Pro Lys Ala Asp Ala Gln
                   85                  90                  95
   Gln Asn Asn Phe Asn Lys Asp Gln Gln Ser Ala Phe Tyr Glu Ile Leu
               100                 105                 110
   Asn Met Pro Asn Leu Asn Glu Ala Gln Arg Asn Gly Phe Ile Gln Ser
           115                 120                 125
   Leu Lys Asp Asp Pro Ser Gln Ser Thr Asn Val Leu Gly Glu Ala Lys
       130                 135                 140
   Lys Leu Asn Glu Ser Gln Ala Pro Lys Ala Asp Asn Asn Phe Asn Lys
   145                 150                 155                 160
   Glu Gln Gln Asn Ala Phe Tyr Glu Ile Leu Asn Met Pro Asn Leu Asn
                   165                 170                 175
   Glu Glu Gln Arg Asn Gly Phe Ile Gln Ser Leu Lys Asp Asp Pro Ser
               180                 185                 190
   Gln Ser Ala Asn Leu Leu Ser Glu Ala Lys Lys Leu Asn Glu Ser Gln
           195                 200                 205
   Ala Pro Lys Ala Asp Asn Lys Phe Asn Lys Glu Gln Gln Asn Ala Phe
       210                 215                 220
   Tyr Glu Ile Leu His Leu Pro Asn Leu Asn Glu Glu Gln Arg Asn Gly
   225                 230                 235                 240
   Phe Ile Gln Ser Leu Lys Asp Asp Pro Ser Val Ser Lys Glu Ile Leu
                   245                 250                 255
   Ala Glu Ala Lys Lys Leu Asn Asp Ala Gln Ala Pro Lys Glu Glu Asp
               260                 265                 270
   Asn Lys Lys Pro Gly Lys Glu Asp Gly Asn Lys Pro Gly Lys Glu Asp
           275                 280                 285
   Gly Asn Lys Pro Gly Lys Glu Asp Asn Lys Lys Pro Gly Lys Glu Asp
       290                 295                 300
   Gly Asn Lys Pro Gly Lys Glu Asp Asn Asn Lys Pro Gly Lys Glu Asp
   305                 310                 315                 320
   Gly Asn Lys Pro Gly Lys Glu Asp Asn Asn Lys Pro Gly Lys Glu Asp
                   325                 330                 335
   Gly Asn Lys Pro Gly Lys Glu Asp Gly Asn Lys Pro Gly Lys Glu Asp
               340                 345                 350
   Gly Asn Gly Val His Val Val Lys Pro Gly Asp Thr Val Asn Asp Ile
           355                 360                 365
   Ala Lys Ala Asn Gly Thr Thr Ala Asp Lys Ile Ala Ala Asp Asn Lys
       370                 375                 380
   Leu Ala Asp Lys Asn Met Ile Lys Pro Gly Gln Glu Leu Val Val Asp
   385                 390                 395                 400
   Lys Lys Gln Pro Ala Asn His Ala Asp Ala Asn Lys Ala Gln Ala Leu
                   405                 410                 415
   Pro Glu Thr Gly Glu Glu Asn Pro Phe Ile Gly Thr Thr Val Phe Gly
               420                 425                 430
   Gly Leu Ser Leu Ala Leu Gly Ala Ala Leu Leu Ala Gly Arg Arg Arg
           435                 440                 445
   Glu Leu
       450
<210> 9
   <211> 415
   <212> PRT
   <213> Staphylococcus aureus
<400> 9
   Met Ser Tyr His Trp Phe Lys Lys Met Leu Leu Ser Thr Ser Ile Leu
   1 5 10 15
   Ile Leu Ser Ser Ser Ser Leu Gly Leu Ala Thr His Thr Val Glu Ala
   20 25 30
   Lys Asp Asn Leu Asn Gly Glu Lys Pro Thr Thr Asn Leu Asn His Asn
   35 40 45
   Ile Thr Ser Pro Ser Val Asn Ser Glu Met Asn Asn Asn Glu Thr Gly
   50 55 60
   Thr Pro His Glu Ser Asn Gln Thr Gly Asn Glu Gly Thr Gly Ser Asn
   65 70 75 80
   Ser Arg Asp Ala Asn Pro Asp Ser Asn Asn Val Lys Pro Asp Ser Asn
   85 90 95
   Asn Gln Asn Pro Ser Thr Asp Ser Lys Pro Asp Pro Asn Asn Gln Asn
   100 105 110
   Pro Ser Pro Asn Pro Lys Pro Asp Pro Asp Asn Pro Lys Pro Lys Pro
   115 120 125
   Asp Pro Asp Lys Pro Lys Pro Asn Leu Asp Pro Lys Pro Asp Pro Asp
   130 135 140
   Asn Pro Lys Pro Lys Pro Asp Pro Lys Pro Asp Pro Asp Lys Pro Lys
   145 150 155 160
   Pro Asn Pro Asp Pro Lys Pro Asp Pro Asp Lys Pro Lys Pro Asn Pro
   165 170 175
   Asn Pro Lys Pro Asp Pro Asn Lys Pro Asn Pro Asn Pro Ser Pro Asp
   180 185 190
   Pro Asp Gln Pro Gly Asp Ser Asn His Ser Gly Gly Ser Lys Asn Gly
   195 200 205
   Gly Thr Trp Asn Pro Asn Ala Ser Asp Gly Ser Asn Gln Gly Gln Trp
   210 215 220
   Gln Pro Asn Gly Asn Gln Gly Asn Ser Gln Asn Pro Thr Gly Asn Asp
   225 230 235 240
   Phe Val Ser Gln Arg Phe Leu Ala Leu Ala Asn Gly Ala Tyr Lys Tyr
   245 250 255
   Asn Pro Tyr Ile Leu Asn Gln Ile Asn Lys Leu Gly Lys Asp Tyr Gly
   260 265 270
   Glu Val Thr Asp Glu Asp Ile Tyr Asn Ile Ile Arg Lys Gln Asn Phe
   275 280 285
   Ser Gly Asn Ala Tyr Leu Asn Gly Leu Gln Gln Gln Ser Asn Tyr Phe
   290 295 300
   Arg Phe Gln Tyr Phe Asn Pro Leu Lys Ser Glu Arg Tyr Tyr Arg Asn
   305 310 315 320
   Leu Asp Glu Gln Val Leu Ala Leu Ile Thr Gly Glu Ile Gly Ser Met
   325 330 335
   Pro Asp Leu Lys Lys Pro Glu Asp Lys Pro Asp Ser Lys Gln Arg Ser
   340 345 350
   Phe Glu Pro His Glu Lys Asp Asp Phe Thr Val Val Lys Lys Gln Glu
   355 360 365
   Asp Asn Lys Lys Ser Ala Ser Thr Ala Tyr Ser Lys Ser Trp Leu Ala
   370 375 380
   Ile Val Cys Ser Met Met Val Val Phe Ser Ile Met Leu Phe Leu Phe
   385 390 395 400
   Val Lys Arg Asn Lys Lys Lys Asn Lys Asn Glu Ser Gln Arg Arg
   405 410 415
<210> 10
   <211> 646
   <212> PRT
   <213> Staphylococcus aureus
<400> 10
   Met Ser Ser Gln Lys Lys Lys Ile Ser Leu Phe Ala Phe Phe Leu Leu
   1 5 10 15
   Thr Val Ile Thr Ile Thr Leu Lys Thr Tyr Phe Ser Tyr Tyr Val Asp
   20 25 30
   Phe Ser Leu Gly Val Lys Gly Leu Val Gln Asn Leu Ile Leu Leu Met
   35 40 45
   Asn Pro Tyr Ser Leu Val Ala Leu Val Leu Ser Val Phe Leu Phe Phe
   50 55 60
   Lys Gly Lys Lys Ala Phe Trp Phe Met Phe Ile Gly Gly Phe Leu Leu
   65 70 75 80
   Thr Phe Leu Leu Tyr Ala Asn Val Val Tyr Phe Arg Phe Phe Ser Asp
   85 90 95
   Phe Leu Thr Phe Ser Thr Leu Asn Gln Val Gly Asn Val Glu Ser Met
   100 105 110
   Gly Gly Ala Val Ser Ala Ser Phe Lys Trp Tyr Asp Phe Val Tyr Phe
   115 120 125
   Ile Asp Thr Leu Val Tyr Leu Phe Ile Leu Ile Phe Lys Thr Lys Trp
   130 135 140
   Leu Asp Thr Lys Ala Phe Ser Lys Lys Phe Val Pro Val Val Met Ala
   145 150 155 160
   Ala Ser Val Ala Leu Phe Phe Leu Asn Leu Ala Phe Ala Glu Thr Asp
   165 170 175
   Arg Pro Glu Leu Leu Thr Arg Thr Phe Asp His Lys Tyr Leu Val Lys
   180 185 190
   Tyr Leu Gly Pro Tyr Asn Phe Thr Val Tyr Asp Gly Val Lys Thr Ile
   195 200 205
   Glu Asn Asn Gln Gln Lys Ala Leu Ala Ser Glu Asp Asp Leu Thr Lys
   210 215 220
   Val Leu Asn Tyr Thr Lys Gln Arg Gln Thr Glu Pro Asn Pro Glu Tyr
   225 230 235 240
   Tyr Gly Val Ala Lys Lys Lys Asn Ile Ile Lys Ile His Leu Glu Ser
   245 250 255
   Phe Gln Thr Phe Leu Ile Asn Lys Lys Val Asn Gly Lys Glu Val Thr
   260 265 270
   Pro Phe Leu Asn Lys Leu Ser Ser Gly Lys Glu Gln Phe Thr Tyr Phe
   275 280 285
   Pro Asn Phe Phe His Gln Thr Gly Gln Gly Lys Thr Ser Asp Ser Glu
   290 295 300
   Phe Thr Met Asp Asn Ser Leu Tyr Gly Leu Pro Gln Gly Ser Ala Phe
   305 310 315 320
   Ser Leu Lys Gly Asp Asn Thr Tyr Gln Ser Leu Pro Ala Ile Leu Asp
   325 330 335
   Gln Lys Gln Gly Tyr Lys Ser Asp Val Met His Gly Asp Tyr Lys Thr
   340 345 350
   Phe Trp Asn Arg Asp Gln Val Tyr Lys His Phe Gly Ile Asp Lys Phe
   355 360 365
   Tyr Asp Ala Thr Tyr Tyr Asp Met Ser Asp Lys Asn Val Val Asn Leu
   370 375 380
   Gly Leu Lys Asp Lys Ile Phe Phe Lys Asp Ser Ala Asn Tyr Gln Ala
   385 390 395 400
   Lys Met Lys Ser Pro Phe Tyr Ser His Leu Ile Thr Leu Thr Asn His
   405 410 415
   Tyr Pro Phe Thr Leu Asp Glu Lys Asp Ala Thr Ile Glu Lys Ser Asn
   420 425 430
   Thr Gly Asp Ala Thr Val Asp Gly Tyr Ile Gln Thr Ala Arg Tyr Leu
   435 440 445
   Asp Glu Ala Leu Glu Glu Tyr Ile Asn Asp Leu Lys Lys Lys Gly Leu
   450 455 460
   Tyr Asp Asn Ser Val Ile Met Ile Tyr Gly Asp His Tyr Gly Ile Ser
   465 470 475 480
   Glu Asn His Asn Asn Ala Met Glu Lys Leu Leu Gly Glu Lys Ile Thr
   485 490 495
   Pro Ala Lys Phe Thr Asp Leu Asn Arg Thr Gly Phe Trp Ile Lys Ile
   500 505 510
   Pro Gly Lys Ser Gly Gly Ile Asn Asn Glu Tyr Ala Gly Gln Val Asp
   515 520 525
   Val Met Pro Thr Ile Leu His Leu Ala Gly Ile Asp Thr Lys Asn Tyr
   530 535 540
   Leu Met Phe Gly Thr Asp Leu Phe Ser Lys Gly His Asn Gln Val Val
   545 550 555 560
   Pro Phe Arg Asn Gly Asp Phe Ile Thr Lys Asp Tyr Lys Tyr Val Asn
   565 570 575
   Gly Lys Ile Tyr Ser Asn Lys Asn Asn Glu Leu Ile Thr Thr Gln Pro
   580 585 590
   Ala Asp Phe Glu Lys Asn Lys Lys Gln Val Glu Lys Asp Leu Glu Met
   595 600 605
   Ser Asp Asn Val Leu Asn Gly Asp Leu Phe Arg Phe Tyr Lys Asn Pro
   610 615 620
   Asp Phe Lys Lys Val Asn Pro Ser Lys Tyr Lys Tyr Glu Thr Gly Pro
   625 630 635 640
   Lys Ala Asn Ser Lys Lys
   645
<210> 11
   <211> 404
   <212> PRT
   <213> Staphylococcus aureus
<400> 11
   Met Phe Ala Ala Leu Leu Gln Ile Lys Asn Tyr Lys Leu Phe Val Ala
   1 5 10 15
   Asn Met Phe Leu Leu Gly Met Gly Ile Ala Val Thr Val Pro Tyr Leu
   20 25 30
   Val Leu Phe Ala Thr Lys Asp Leu Gly Met Thr Thr Asn Gln Tyr Gly
   35 40 45
   Leu Leu Leu Ala Ser Ala Ala Ile Ser Gln Phe Thr Val Asn Ser Ile
   50 55 60
   Ile Ala Arg Phe Ser Asp Thr His His Phe Asn Arg Lys Ile Ile Ile
   65 70 75 80
   Ile Leu Ala Leu Leu Met Gly Ala Leu Gly Phe Ser Ile Tyr Phe Phe
   85 90 95
   Val Asp Thr Ile Trp Leu Phe Ile Leu Leu Tyr Ala Ile Phe Gln Gly
   100 105 110
   Leu Phe Ala Pro Ala Met Pro Gln Leu Tyr Ala Ser Ala Arg Glu Ser
   115 120 125
   Ile Asn Val Ser Ser Ser Lys Asp Arg Ala Gln Phe Ala Asn Thr Val
   130 135 140
   Leu Arg Ser Met Phe Ser Leu Gly Phe Leu Phe Gly Pro Phe Ile Gly
   145 150 155 160
   Ala Gln Leu Ile Gly Leu Lys Gly Tyr Ala Gly Leu Phe Gly Gly Thr
   165 170 175
   Ile Ser Ile Ile Leu Phe Thr Leu Val Leu Gln Val Phe Phe Tyr Lys
   180 185 190
   Asp Leu Asn Ile Lys His Pro Ile Ser Thr Gln Gln His Val Glu Lys
   195 200 205
   Ile Ala Pro Asn Met Phe Lys Asp Lys Thr Leu Leu Leu Pro Phe Ile
   210 215 220
   Ala Phe Ile Leu Leu His Ile Gly Gln Trp Met Tyr Thr Met Asn Met
   225 230 235 240
   Pro Leu Phe Val Thr Asp Tyr Leu Lys Glu Asn Glu Gln His Val Gly
   245 250 255
   Tyr Leu Ala Ser Leu Cys Ala Gly Leu Glu Val Pro Phe Met Ile Ile
   260 265 270
   Leu Gly Val Leu Ser Ser Arg Leu His Thr Arg Thr Leu Leu Ile Tyr
   275 280 285
   Gly Ala Ile Phe Gly Gly Leu Phe Tyr Phe Ser Ile Gly Val Phe Lys
   290 295 300
   Asn Phe Tyr Met Met Leu Ala Gly Gln Val Phe Leu Ala Ile Phe Leu
   305 310 315 320
   Ala Val Leu Leu Gly Ile Gly Ile Ser Tyr Phe Gln Asp Ile Leu Pro
   325 330 335
   Asp Phe Pro Gly Tyr Ala Ser Thr Leu Phe Ser Asn Ala Met Val Ile
   340 345 350
   Gly Gln Leu Gly Gly Asn Leu Leu Gly Gly Ala Met Ser His Trp Val
   355 360 365
   Gly Leu Glu Asn Val Phe Phe Val Ser Ala Ala Ser Ile Met Leu Gly
   370 375 380
   Met Ile Leu Ile Phe Phe Thr Lys Asn Gln Lys Ile Thr Lys Glu Asp
   385 390 395 400
   Met Ile Ser Thr
<210> 12
   <211> 336
   <212> PRT
   <213> Staphylococcus aureus
<400> 12
   Met Arg Ala Ala Val Val Thr Lys Asp His Lys Val Ser Ile Glu Asp
   1               5                   10                  15
   Lys Lys Leu Arg Ala Leu Lys Pro Gly Glu Ala Leu Val Gln Thr Glu
               20                  25                  30
   Tyr Cys Gly Val Cys His Thr Asp Leu His Val Lys Asn Ala Asp Phe
           35                  40                  45
   Gly Asp Val Thr Gly Val Thr Leu Gly His Glu Gly Ile Gly Lys Val
       50                  55                  60
   Ile Glu Val Ala Glu Asp Val Glu Ser Leu Lys Ile Gly Asp Arg Val
   65                  70                  75                  80
   Ser Ile Ala Trp Met Phe Glu Ser Cys Gly Arg Cys Glu Tyr Cys Thr
                   85                  90                  95
   Thr Gly Arg Glu Thr Leu Cys Arg Ser Val Lys Asn Ala Gly Tyr Thr
               100                 105                 110
   Val Asp Gly Ala Met Ala Glu Gln Val Ile Val Thr Ala Asp Tyr Ala
           115                 120                 125
   Val Lys Val Pro Glu Lys Leu Asp Pro Ala Ala Ala Ser Ser Ile Thr
       130                 135                 140
   Cys Ala Gly Val Thr Thr Tyr Lys Ala Val Lys Val Ser Asn Val Lys
   145                 150                 155                 160
   Pro Gly Gln Trp Leu Gly Val Phe Gly Ile Gly Gly Leu Gly Asn Leu
                   165                 170                 175
   Ala Leu Gln Tyr Ala Lys Asn Val Met Gly Ala Lys Ile Val Ala Phe
               180                 185                 190
   Asp Ile Asn Asp Asp Lys Leu Ala Phe Ala Lys Glu Leu Gly Ala Asp
           195                 200                 205
   Ala Ile Ile Asn Ser Lys Asp Val Asp Pro Val Ala Glu Val Met Lys
       210                 215                 220
   Leu Thr Asp Asn Lys Gly Leu Asp Ala Thr Val Val Thr Ser Val Ala
   225                 230                 235                 240
   Lys Thr Pro Phe Asn Gln Ala Val Asp Val Val Lys Ala Gly Ala Arg
   245 250 255
   Val Val Ala Val Gly Leu Pro Val Asp Lys Met Asn Leu Asp Ile Pro
   260 265 270
   Arg Leu Val Leu Asp Gly Ile Glu Val Val Gly Ser Leu Val Gly Thr
   275 280 285
   Arg Gln Asp Leu Arg Glu Ala Phe Glu Phe Ala Ala Glu Asn Lys Val
   290 295 300
   Thr Pro Lys Val Gln Leu Arg Lys Leu Glu Glu Ile Asn Asp Ile Phe
   305 310 315 320
   Glu Glu Met Glu Lys Gly Thr Ile Thr Gly Arg Met Val Ile Lys Phe
   325 330 335
<210> 13
   <211> 393
   <212> PRT
   <213> Staphylococcus aureus
<400> 13
   Met Asn Ser Ser Tyr Lys Ser Arg Val Phe Asn Ile Ile Ser Ile Ile
   1               5                   10                  15
   Met Val Ser Met Leu Ile Leu Ser Leu Gly Ala Phe Ala Asn Asn Asn
               20                  25                  30
   Lys Ala Lys Ala Asp Ser His Ser Lys Gln Leu Glu Ile Asn Val Lys
           35                  40                  45
   Ser Asp Lys Val Pro Gln Lys Val Lys Asp Leu Ala Gln Gln Gln Phe
       50                  55                  60
   Ala Gly Tyr Ala Lys Ala Leu Asp Lys Gln Ser Asn Ala Lys Thr Gly
   65                  70                  75                  80
   Lys Tyr Glu Leu Gly Glu Ala Phe Lys Ile Tyr Lys Phe Asn Gly Glu
                   85                  90                  95
   Glu Asp Asn Ser Tyr Tyr Tyr Pro Val Ile Lys Asp Gly Lys Ile Val
               100                 105                 110
   Tyr Thr Leu Thr Leu Ser Pro Lys Asn Lys Asp Asp Leu Asn Lys Ser
           115                 120                 125
   Lys Glu Asp Met Asn Tyr Ser Val Lys Ile Ser Asn Phe Ile Ala Lys
   130 135 140
   Asp Leu Asp Gln Ile Lys Asp Lys Asn Ser Asn Ile Thr Val Leu Thr
   145 150 155 160
   Asp Glu Lys Gly Phe Tyr Phe Glu Glu Asp Gly Lys Val Arg Leu Val
   165 170 175
   Lys Ala Thr Pro Leu Pro Gly Asn Val Lys Glu Lys Glu Ser Ala Lys
   180 185 190
   Thr Val Ser Ala Lys Leu Lys Gln Glu Leu Lys Asn Thr Val Thr Pro
   195 200 205
   Thr Lys Val Glu Glu Asn Glu Ala Ile Gln Glu Asp Gln Val Gln Tyr
   210 215 220
   Glu Asn Thr Leu Lys Asn Phe Lys Ile Arg Glu Gln Gln Phe Asp Asn
   225 230 235 240
   Ser Trp Cys Ala Gly Phe Ser Met Ala Ala Leu Leu Asn Ala Thr Lys
   245 250 255
   Asn Thr Asp Thr Tyr Asn Ala His Asp Ile Met Arg Thr Leu Tyr Pro
   260 265 270
   Glu Val Ser Glu Gln Asp Leu Pro Asn Cys Ser Thr Phe Pro Asn Gln
   275 280 285
   Met Ile Glu Tyr Gly Lys Ser Gln Gly Arg Asp Ile His Tyr Gln Glu
   290 295 300
   Gly Val Pro Ser Tyr Glu Gln Val Asp Gln Leu Thr Lys Asp Asn Val
   305 310 315 320
   Gly Ile Met Ile Leu Ala Gln Ser Val Ser Gln Asn Pro Asn Asp Pro
   325 330 335
   His Leu Gly His Ala Leu Ala Val Val Gly Asn Ala Lys Ile Asn Asp
   340 345 350
   Gln Glu Lys Leu Ile Tyr Trp Asn Pro Trp Asp Thr Glu Leu Ser Ile
   355 360 365
   Gln Asp Ala Asp Ser Ser Leu Leu His Leu Ser Phe Asn Arg Asp Tyr
   370 375 380
   Asn Trp Tyr Gly Ser Met Ile Gly Tyr
   385 390
<210> 14
   <211> 315
   <212> PRT
   <213> Staphylococcus aureus
<400> 14
   Met Leu Lys Asn Lys Ile Leu Ala Thr Thr Leu Ser Val Ser Leu Leu
   1               5                   10                  15
   Ala Pro Leu Ala Asn Pro Leu Leu Glu Asn Ala Lys Ala Ala Asn Asp
               20                  25                  30
   Thr Glu Asp Ile Gly Lys Gly Ser Asp Ile Glu Ile Ile Lys Arg Thr
           35                  40                  45
   Glu Asp Lys Thr Ser Asn Lys Trp Gly Val Thr Gln Asn Ile Gln Phe
       50                  55                  60
   Asp Phe Val Lys Asp Lys Lys Tyr Asn Lys Asp Ala Leu Ile Leu Lys
   65                  70                  75                  80
   Met Gln Gly Phe Ile Ser Ser Arg Thr Thr Tyr Tyr Asn Tyr Lys Lys
                   85                  90                  95
   Thr Asn His Val Lys Ala Met Arg Trp Pro Phe Gln Tyr Asn Ile Gly
               100                 105                 110
   Leu Lys Thr Asn Asp Lys Tyr Val Ser Leu Ile Asn Tyr Leu Pro Lys
           115                 120                 125
   Asn Lys Ile Glu Ser Thr Asn Val Ser Gln Thr Leu Gly Tyr Asn Ile
       130                 135                 140
   Gly Gly Asn Phe Gln Ser Ala Pro Ser Leu Gly Gly Asn Gly Ser Phe
   145                 150                 155                 160
   Asn Tyr Ser Lys Ser Ile Ser Tyr Thr Gln Gln Asn Tyr Val Ser Glu
                   165                 170                 175
   Val Glu Gln Gln Asn Ser Lys Ser Val Leu Trp Gly Val Lys Ala Asn
               180                 185                 190
   Ser Phe Ala Thr Glu Ser Gly Gln Lys Ser Ala Phe Asp Ser Asp Leu
           195                 200                 205
   Phe Val Gly Tyr Lys Pro His Ser Lys Asp Pro Arg Asp Tyr Phe Val
       210                 215                 220
   Pro Asp Ser Glu Leu Pro Pro Leu Val Gln Ser Gly Phe Asn Pro Ser
   225 230 235 240
   Phe Ile Ala Thr Val Ser His Glu Lys Gly Ser Ser Asp Thr Ser Glu
   245 250 255
   Phe Glu Ile Thr Tyr Gly Arg Asn Met Asp Val Thr His Ala Ile Lys
   260 265 270
   Arg Ser Thr His Tyr Gly Asn Ser Tyr Leu Asp Gly His Arg Val His
   275 280 285
   Asn Ala Phe Val Asn Arg Asn Tyr Thr Val Lys Tyr Glu Val Asn Trp
   290 295 300
   Lys Thr His Glu Ile Lys Val Lys Gly Gln Asn
   305 310 315
<210> 15
   <211> 309
   <212> PRT
   <213> Staphylococcus aureus
<400> 15
   Met Ile Lys Asn Lys Ile Leu Thr Ala Thr Leu Ala Val Gly Leu Ile
   1               5                   10                  15
   Ala Pro Leu Ala Asn Pro Phe Ile Glu Ile Ser Lys Ala Glu Asn Lys
               20                  25                  30
   Ile Glu Asp Ile Gly Gln Gly Ala Glu Ile Ile Lys Arg Thr Gln Asp
           35                  40                  45
   Ile Thr Ser Lys Arg Leu Ala Ile Thr Gln Asn Ile Gln Phe Asp Phe
       50                  55                  60
   Val Lys Asp Lys Lys Tyr Asn Lys Asp Ala Leu Val Val Lys Met Gln
   65                  70                  75                  80
   Gly Phe Ile Ser Ser Arg Thr Thr Tyr Ser Asp Leu Lys Lys Tyr Pro
                   85                  90                  95
   Tyr Ile Lys Arg Met Ile Trp Pro Phe Gln Tyr Asn Ile Ser Leu Lys
               100                 105                 110
   Thr Lys Asp Ser Asn Val Asp Leu Ile Asn Tyr Leu Pro Lys Asn Lys
           115                 120                 125
   Ile Asp Ser Ala Asp Val Ser Gln Lys Leu Gly Tyr Asn Ile Gly Gly
       130                 135                 140
   Asn Phe Gln Ser Ala Pro Ser Ile Gly Gly Ser Gly Ser Phe Asn Tyr
   145 150 155 160
   Ser Lys Thr Ile Ser Tyr Asn Gln Lys Asn Tyr Val Thr Glu Val Glu
   165 170 175
   Ser Gln Asn Ser Lys Gly Val Lys Trp Gly Val Lys Ala Asn Ser Phe
   180 185 190
   Val Thr Pro Asn Gly Gln Val Ser Ala Tyr Asp Gln Tyr Leu Phe Ala
   195 200 205
   Gln Asp Pro Thr Gly Pro Ala Ala Arg Asp Tyr Phe Val Pro Asp Asn
   210 215 220
   Gln Leu Pro Pro Leu Ile Gln Ser Gly Phe Asn Pro Ser Phe Ile Thr
   225 230 235 240
   Thr Leu Ser His Glu Arg Gly Lys Gly Asp Lys Ser Glu Phe Glu Ile
   245 250 255
   Thr Tyr Gly Arg Asn Met Asp Ala Thr Tyr Ala Tyr Val Thr Arg His
   260 265 270
   Arg Leu Ala Val Asp Arg Lys His Asp Ala Phe Lys Asn Arg Asn Val
   275 280 285
   Thr Val Lys Tyr Glu Val Asn Trp Lys Thr His Glu Val Lys Ile Lys
   290 295 300
   Ser Ile Thr Pro Lys
   305
<210> 16
   <211> 319
   <212> PRT
   <213> Staphylococcus aureus
<400> 16
   Met Lys Thr Arg Ile Val Ser Ser Val Thr Thr Thr Leu Leu Leu Gly
   1 5 10 15
   Ser Ile Leu Met Asn Pro Val Ala Asn Ala Ala Asp Ser Asp Ile Asn
   20 25 30
   Ile Lys Thr Gly Thr Thr Asp Ile Gly Ser Asn Thr Thr Val Lys Thr
   35 40 45
   Gly Asp Leu Val Thr Tyr Asp Lys Glu Asn Gly Met His Lys Lys Val
       50                  55                  60
   Phe Tyr Ser Phe Ile Asp Asp Lys Asn His Asn Lys Lys Leu Leu Val
   65                  70                  75                  80
   Ile Arg Thr Lys Gly Thr Ile Ala Gly Gln Tyr Arg Val Tyr Ser Glu
                   85                  90                  95
   Glu Gly Ala Asn Lys Ser Gly Leu Ala Trp Pro Ser Ala Phe Lys Val
               100                 105                 110
   Gln Leu Gln Leu Pro Asp Asn Glu Val Ala Gln Ile Ser Asp Tyr Tyr
           115                 120                 125
   Pro Arg Asn Ser Ile Asp Thr Lys Glu Tyr Met Ser Thr Leu Thr Tyr
       130                 135                 140
   Gly Phe Asn Gly Asn Val Thr Gly Asp Asp Thr Gly Lys Ile Gly Gly
   145                 150                 155                 160
   Leu Ile Gly Ala Asn Val Ser Ile Gly His Thr Leu Lys Tyr Val Gln
                   165                 170                 175
   Pro Asp Phe Lys Thr Ile Leu Glu Ser Pro Thr Asp Lys Lys Val Gly
               180                 185                 190
   Trp Lys Val Ile Phe Asn Asn Met Val Asn Gln Asn Trp Gly Pro Tyr
           195                 200                 205
   Asp Arg Asp Ser Trp Asn Pro Val Tyr Gly Asn Gln Leu Phe Met Lys
       210                 215                 220
   Thr Arg Asn Gly Ser Met Lys Ala Ala Glu Asn Phe Leu Asp Pro Asn
   225                 230                 235                 240
   Lys Ala Ser Ser Leu Leu Ser Ser Gly Phe Ser Pro Asp Phe Ala Thr
                   245                 250                 255
   Val Ile Thr Met Asp Arg Lys Ala Ser Lys Gln Gln Thr Asn Ile Asp
               260                 265                 270
   Val Ile Tyr Glu Arg Val Arg Asp Asp Tyr Gln Leu His Trp Thr Ser
           275                 280                 285
   Thr Asn Trp Lys Gly Thr Asn Thr Lys Asp Lys Trp Thr Asp Arg Ser
       290                 295                 300
   Ser Glu Arg Tyr Lys Ile Asp Trp Glu Lys Glu Glu Met Thr Asn
   305 310 315
<210> 17
   <211> 328
   <212> PRT
   <213> Staphylococcus aureus
<400> 17
   Met Lys Lys Cys Ile Lys Thr Leu Phe Leu Ser Ile Ile Leu Val Val
   1               5                   10                  15
   Met Gly Gly Trp Tyr His Ser Ala His Ala Ser Asp Ser Leu Ser Lys
               20                  25                  30
   Ser Pro Glu Asn Trp Met Ser Lys Leu Asp Glu Ser Lys His Leu Thr
           35                  40                  45
   Glu Ile Asn Met Pro Gly Ser His Asp Ser Gly Ser Phe Thr Leu Thr
       50                  55                  60
   Asp Pro Val Lys Ser Val Trp Ala Lys Thr Gln Gly Lys Asp Tyr Leu
   65                  70                  75                  80
   Thr Gln Met Lys Ser Gly Val Arg Phe Phe Asp Ile Arg Gly Arg Ala
                   85                  90                  95
   Ser Ala Asp Asn Met Ile Ser Val His His Gly Met Val Tyr Leu His
               100                 105                 110
   His Glu Leu Gly Lys Phe Leu Asp Asp Ala Lys Tyr Tyr Leu Ser Ala
           115                 120                 125
   Tyr Pro Asn Glu Thr Ile Val Met Ser Met Lys Lys Asp Tyr Asp Ser
       130                 135                 140
   Asp Ser Lys Val Thr Lys Thr Phe Glu Glu Ile Phe Arg Glu Tyr Tyr
   145                 150                 155                 160
   Tyr Asn Asn Pro Gln Tyr Gln Asn Leu Phe Tyr Thr Gly Ser Asn Ala
                   165                 170                 175
   Asn Pro Thr Leu Lys Glu Thr Lys Gly Lys Ile Val Leu Phe Asn Arg
               180                 185                 190
   Met Gly Gly Thr Tyr Ile Lys Ser Gly Tyr Gly Ala Asp Thr Ser Gly
           195                 200                 205
   Ile Gln Trp Ala Asp Asn Ala Thr Phe Glu Thr Lys Ile Asn Asn Gly
       210                 215                 220
   Ser Leu Asn Leu Lys Val Gln Asp Glu Tyr Lys Asp Tyr Tyr Asp Lys
   225 230 235 240
   Lys Val Glu Ala Val Lys Asn Leu Leu Ala Lys Ala Lys Thr Asp Ser
   245 250 255
   Asn Lys Asp Asn Val Tyr Val Asn Phe Leu Ser Val Ala Ser Gly Gly
   260 265 270
   Ser Ala Phe Asn Ser Thr Tyr Asn Tyr Ala Ser His Ile Asn Pro Glu
   275 280 285
   Ile Ala Lys Thr Ile Lys Glu Asn Gly Lys Ala Arg Thr Gly Trp Leu
   290 295 300
   Ile Val Asp Tyr Ala Gly Tyr Pro Trp Pro Gly Tyr Asp Asp Ile Val
   305 310 315 320
   Ser Glu Ile Ile Asp Ser Asn Lys
   325
<210> 18
   <211> 338
   <212> PRT
   <213> Staphylococcus aureus
<400> 18
   Met Ile Lys Gln Leu Tyr Lys Asn Ile Thr Ile Cys Ser Leu Ala Ile
   1               5                   10                  15
   Ser Thr Ala Leu Thr Val Phe Pro Ala Thr Ser Tyr Ala Lys Ile Asn
               20                  25                  30
   Ser Glu Ile Lys Ala Val Ser Glu Lys Asn Leu Asp Gly Asp Thr Lys
           35                  40                  45
   Met Tyr Thr Arg Thr Ala Thr Thr Ser Asp Ser Gln Lys Asn Ile Thr
       50                  55                  60
   Gln Ser Leu Gln Phe Asn Phe Leu Thr Glu Pro Asn Tyr Asp Lys Glu
   65                  70                  75                  80
   Thr Val Phe Ile Lys Ala Lys Gly Thr Ile Gly Ser Gly Leu Arg Ile
                   85                  90                  95
   Leu Asp Pro Asn Gly Tyr Trp Asn Ser Thr Leu Arg Trp Pro Gly Ser
               100                 105                 110
   Tyr Ser Val Ser Ile Gln Asn Val Asp Asp Asn Asn Asn Thr Asn Val
   115 120 125
   Thr Asp Phe Ala Pro Lys Asn Gln Asp Glu Ser Arg Glu Val Lys Tyr
   130 135 140
   Thr Tyr Gly Tyr Lys Thr Gly Gly Asp Phe Ser Ile Asn Arg Gly Gly
   145 150 155 160
   Leu Thr Gly Asn Ile Thr Lys Glu Ser Asn Tyr Ser Glu Thr Ile Ser
   165 170 175
   Tyr Gln Gln Pro Ser Tyr Arg Thr Leu Leu Asp Gln Ser Thr Ser His
   180 185 190
   Lys Gly Val Gly Trp Lys Val Glu Ala His Leu Ile Asn Asn Met Gly
   195 200 205
   His Asp His Thr Arg Gln Leu Thr Asn Asp Ser Asp Asn Arg Thr Lys
   210 215 220
   Ser Glu Ile Phe Ser Leu Thr Arg Asn Gly Asn Leu Trp Ala Lys Asp
   225 230 235 240
   Asn Phe Thr Pro Lys Asp Lys Met Pro Val Thr Val Ser Glu Gly Phe
   245 250 255
   Asn Pro Glu Phe Leu Ala Val Met Ser His Asp Lys Lys Asp Lys Gly
   260 265 270
   Lys Ser Gln Phe Val Val His Tyr Lys Arg Ser Met Asp Glu Phe Lys
   275 280 285
   Ile Asp Trp Asn Arg His Gly Phe Trp Gly Tyr Trp Ser Gly Glu Asn
   290 295 300
   His Val Asp Lys Lys Glu Glu Lys Leu Ser Ala Leu Tyr Glu Val Asp
   305 310 315 320
   Trp Lys Thr His Asp Val Lys Phe Val Lys Val Leu Asn Asp Asn Glu
   325 330 335
   Lys Lys
<210> 19
   <211> 260
   <212> PRT
   <213> Staphylococcus aureus
<400> 19
   Met Ser Lys Met Lys Lys Thr Ala Phe Thr Leu Leu Leu Phe Ile Ala
   1               5                   10                  15
   Leu Thr Leu Thr Thr Ser Pro Leu Val Asn Gly Ser Glu Lys Ser Glu
               20                  25                  30
   Glu Ile Asn Glu Lys Asp Leu Arg Lys Lys Ser Glu Leu Gln Gly Thr
           35                  40                  45
   Ala Leu Gly Asn Leu Lys Gln Ile Tyr Tyr Tyr Asn Glu Lys Ala Lys
       50                  55                  60
   Thr Glu Asn Lys Glu Ser His Asp Gln Phe Leu Gln His Thr Ile Leu
   65                  70                  75                  80
   Phe Lys Gly Phe Phe Thr Asp His Ser Trp Tyr Asn Asp Leu Leu Val
                   85                  90                  95
   Asp Phe Asp Ser Lys Asp Ile Val Asp Lys Tyr Lys Gly Lys Lys Val
               100                 105                 110
   Asp Leu Tyr Gly Ala Tyr Tyr Gly Tyr Gln Cys Ala Gly Gly Thr Pro
           115                 120                 125
   Asn Lys Thr Ala Cys Met Tyr Gly Gly Val Thr Leu His Asp Asn Asn
       130                 135                 140
   Arg Leu Thr Glu Glu Lys Lys Val Pro Ile Asn Leu Trp Leu Asp Gly
   145                 150                 155                 160
   Lys Gln Asn Thr Val Pro Leu Glu Thr Val Lys Thr Asn Lys Lys Asn
                   165                 170                 175
   Val Thr Val Gln Glu Leu Asp Leu Gln Ala Arg Arg Tyr Leu Gln Glu
               180                 185                 190
   Lys Tyr Asn Leu Tyr Asn Ser Asp Val Phe Asp Gly Lys Val Gln Arg
           195                 200                 205
   Gly Leu Ile Val Phe His Thr Ser Thr Glu Pro Ser Val Asn Tyr Asp
       210                 215                 220
   Leu Phe Gly Ala Gln Gly Gln Tyr Ser Asn Thr Leu Leu Arg Ile Tyr
   225                 230                 235                 240
   Arg Asp Asn Lys Thr Ile Asn Ser Glu Asn Met His Ile Asp Ile Tyr
                   245                 250                 255
   Leu Tyr Thr Ser
   260
<210> 20
   <211> 231
   <212> PRT
   <213> Staphylococcus aureus
<400> 20
   Met Lys Lys Thr Leu Leu Ala Ser Ser Leu Ala Val Gly Leu Gly Ile
   1               5                   10                  15
   Val Ala Gly Asn Ala Gly His Glu Ala His Ala Ser Glu Ala Asp Leu
               20                  25                  30
   Asn Lys Ala Ser Leu Ala Gln Met Ala Gln Ser Asn Asp Gln Thr Leu
           35                  40                  45
   Asn Gln Lys Pro Ile Glu Ala Gly Ala Tyr Asn Tyr Thr Phe Asp Tyr
       50                  55                  60
   Glu Gly Phe Thr Tyr His Phe Glu Ser Asp Gly Thr His Phe Ala Trp
   65                  70                  75                  80
   Asn Tyr His Ala Thr Gly Ala Asn Gly Ala Asn Met Ser Ala Gln Ala
                   85                  90                  95
   Pro Ala Thr Asn Asn Val Glu Pro Ser Ala Val Gln Ala Asn Gln Val
               100                 105                 110
   Gln Ser Gln Glu Val Glu Ala Pro Gln Asn Ala Gln Thr Gln Gln Pro
           115                 120                 125
   Gln Ala Ser Thr Ser Asn Asn Ser Gln Val Thr Ala Thr Pro Thr Glu
       130                 135                 140
   Ser Lys Ala Ser Glu Gly Ser Ser Val Asn Val Asn Ala His Leu Lys
   145                 150                 155                 160
   Gln Ile Ala Gln Arg Glu Ser Gly Gly Asn Ile His Ala Val Asn Pro
                   165                 170                 175
   Thr Ser Gly Ala Ala Gly Lys Tyr Gln Phe Leu Gln Ser Thr Trp Asp
               180                 185                 190
   Ser Val Ala Pro Ala Lys Tyr Lys Gly Val Ser Pro Ala Asn Ala Pro
           195                 200                 205
   Glu Ser Val Gln Asp Ala Ala Ala Val Lys Leu Tyr Asn Thr Gly Gly
       210                 215                 220
   Ala Gly His Trp Val Thr Ala
   225                 230
<210> 21
   <211> 342
   <212> PRT
   <213> Staphylococcus aureus
<400> 21
   Met Lys Gly Lys Phe Leu Lys Val Ser Ser Leu Phe Val Ala Thr Leu
   1               5                   10                  15
   Thr Thr Ala Thr Leu Val Ser Ser Pro Ala Ala Asn Ala Leu Ser Ser
               20                  25                  30
   Lys Ala Met Asp Asn His Pro Gln Gln Thr Gln Ser Ser Lys Gln Gln
           35                  40                  45
   Thr Pro Lys Ile Lys Lys Gly Gly Asn Leu Lys Pro Leu Glu Gln Arg
       50                  55                  60
   Glu His Ala Asn Val Ile Leu Pro Asn Asn Asp Arg His Gln Ile Thr
   65                  70                  75                  80
   Asp Thr Thr Asn Gly His Tyr Ala Pro Val Thr Tyr Ile Gln Val Glu
                   85                  90                  95
   Ala Pro Thr Gly Thr Phe Ile Ala Ser Gly Val Val Val Gly Lys Asp
               100                 105                 110
   Thr Leu Leu Thr Asn Lys His Val Val Asp Ala Thr His Gly Asp Pro
           115                 120                 125
   His Ala Leu Lys Ala Phe Pro Ser Ala Ile Asn Gln Asp Asn Tyr Pro
       130                 135                 140
   Asn Gly Gly Phe Thr Ala Glu Gln Ile Thr Lys Tyr Ser Gly Glu Gly
   145                 150                 155                 160
   Asp Leu Ala Ile Val Lys Phe Ser Pro Asn Glu Gln Asn Lys His Ile
                   165                 170                 175
   Gly Glu Val Val Lys Pro Ala Thr Met Ser Asn Asn Ala Glu Thr Gln
               180                 185                 190
   Val Asn Gln Asn Ile Thr Val Thr Gly Tyr Pro Gly Asp Lys Pro Val
           195                 200                 205
   Ala Thr Met Trp Glu Ser Lys Gly Lys Ile Thr Tyr Leu Lys Gly Glu
   210 215 220
   Ala Met Gln Tyr Asp Leu Ser Thr Thr Gly Gly Asn Ser Gly Ser Pro
   225 230 235 240
   Val Phe Asn Glu Lys Asn Glu Val Ile Gly Ile His Trp Gly Gly Val
   245 250 255
   Pro Asn Glu Phe Asn Gly Ala Val Phe Ile Asn Glu Asn Val Arg Asn
   260 265 270
   Phe Leu Lys Gln Asn Ile Glu Asp Ile His Phe Ala Asn Asp Asp Gln
   275 280 285
   Pro Asn Asn Pro Asp Asn Pro Asp Asn Pro Asn Asn Pro Asp Asn Pro
   290 295 300
   Asn Asn Pro Asp Asn Pro Asn Asn Pro Asp Glu Pro Asn Asn Pro Asp
   305 310 315 320
   Asn Pro Asn Asn Pro Asp Asn Pro Asp Asn Gly Asp Asn Asn Asn Ser
   325 330 335
   Asp Asn Pro Asp Ala Ala
   340
<210> 22
   <211> 253
   <212> PRT
   <213> Staphylococcus aureus
<400> 22
   Met Arg Thr Pro Ile Ile Ala Gly Asn Trp Lys Met Asn Lys Thr Val
   1               5                   10                  15
   Gln Glu Ala Lys Asp Phe Val Asn Ala Leu Pro Thr Leu Pro Asp Ser
               20                  25                  30
   Lys Glu Val Glu Ser Val Ile Cys Ala Pro Ala Ile Gln Leu Asp Ala
           35                  40                  45
   Leu Thr Thr Ala Val Lys Glu Gly Lys Ala Gln Gly Leu Glu Ile Gly
       50                  55                  60
   Ala Gln Asn Thr Tyr Phe Glu Asp Asn Gly Ala Phe Thr Gly Glu Thr
   65                  70                  75                  80
   Ser Pro Val Ala Leu Ala Asp Leu Gly Val Lys Tyr Val Val Ile Gly
                   85                  90                  95
   His Ser Glu Arg Arg Glu Leu Phe His Glu Thr Asp Glu Glu Ile Asn
               100                 105                 110
   Lys Lys Ala His Ala Ile Phe Lys His Gly Met Thr Pro Ile Ile Cys
           115                 120                 125
   Val Gly Glu Thr Asp Glu Glu Arg Glu Ser Gly Lys Ala Asn Asp Val
       130                 135                 140
   Val Gly Glu Gln Val Lys Lys Ala Val Ala Gly Leu Ser Glu Asp Gln
   145                 150                 155                 160
   Leu Lys Ser Val Val Ile Ala Tyr Glu Pro Ile Trp Ala Ile Gly Thr
                   165                 170                 175
   Gly Lys Ser Ser Thr Ser Glu Asp Ala Asn Glu Met Cys Ala Phe Val
               180                 185                 190
   Arg Gln Thr Ile Ala Asp Leu Ser Ser Lys Glu Val Ser Glu Ala Thr
           195                 200                 205
   Arg Ile Gln Tyr Gly Gly Ser Val Lys Pro Asn Asn Ile Lys Glu Tyr
       210                 215                 220
   Met Ala Gln Thr Asp Ile Asp Gly Ala Leu Val Gly Gly Ala Ser Leu
   225                 230                 235                 240
   Lys Val Glu Asp Phe Val Gln Leu Leu Glu Gly Ala Lys
                   245                 250
<210> 23
   <211> 233
   <212> PRT
   <213> Staphylococcus aureus
<400> 23
   Met Lys Lys Thr Ile Met Ala Ser Ser Leu Ala Val Ala Leu Gly Val
   1               5                   10                  15
   Thr Gly Tyr Ala Ala Gly Thr Gly His Gln Ala His Ala Ala Glu Val
               20                  25                  30
   Asn Val Asp Gln Ala His Leu Val Asp Leu Ala His Asn His Gln Asp
           35                  40                  45
   Gln Leu Asn Ala Ala Pro Ile Lys Asp Gly Ala Tyr Asp Ile His Phe
       50                  55                  60
   Val Lys Asp Gly Phe Gln Tyr Asn Phe Thr Ser Asn Gly Thr Thr Trp
   65                  70                  75                  80
   Ser Trp Ser Tyr Glu Ala Ala Asn Gly Gln Thr Ala Gly Phe Ser Asn
                   85                  90                  95
   Val Ala Gly Ala Asp Tyr Thr Thr Ser Tyr Asn Gln Gly Ser Asp Val
               100                 105                 110
   Gln Ser Val Ser Tyr Asn Ala Gln Ser Ser Asn Ser Asn Val Glu Ala
           115                 120                 125
   Val Ser Ala Pro Thr Tyr His Asn Tyr Ser Thr Ser Thr Thr Ser Ser
       130                 135                 140
   Ser Val Arg Leu Ser Asn Gly Asn Thr Ala Gly Ala Thr Gly Ser Ser
   145                 150                 155                 160
   Ala Ala Gln Ile Met Ala Gln Arg Thr Gly Val Ser Ala Ser Thr Trp
                   165                 170                 175
   Ala Ala Ile Ile Ala Arg Glu Ser Asn Gly Gln Val Asn Ala Tyr Asn
               180                 185                 190
   Pro Ser Gly Ala Ser Gly Leu Phe Gln Thr Met Pro Gly Trp Gly Pro
           195                 200                 205
   Thr Asn Thr Val Asp Gln Gln Ile Asn Ala Ala Val Lys Ala Tyr Lys
       210                 215                 220
   Ala Gln Gly Leu Gly Ala Trp Gly Phe
   225                 230
<210> 24
   <211> 258
   <212> PRT
   <213> Staphylococcus aureus
<400> 24
   Met Lys Lys Asn Phe Lys Leu Arg Ile Ser Thr Leu Leu Leu Ile Val
   1               5                   10                  15
   Ile Leu Val Val Phe Ala Val Leu Leu Ile Val Asn Glu Thr Lys Leu
               20                  25                  30
   Phe Lys Asn Asp Val Asn Tyr Ser Phe Asp Glu Ala Val Ser Met Gln
           35                  40                  45
   Gln Gly Lys Gly Ile Val Gln Thr Lys Glu Glu Asp Gly Lys Phe Val
       50                  55                  60
   Glu Ala Asn Asn Asn Glu Ile Ala Lys Ala Met Thr Ile Ser His Lys
   65 70 75 80
   Asp Asn Asp Met Lys Tyr Met Asp Ile Thr Glu Lys Val Pro Met Ser
   85 90 95
   Glu Ser Glu Val Asn Gln Leu Leu Lys Gly Lys Gly Ile Leu Glu Asn
   100 105 110
   Arg Gly Lys Val Phe Leu Glu Ala Gln Glu Lys Tyr Glu Val Asn Val
   115 120 125
   Ile Tyr Leu Val Ser His Ala Leu Val Glu Thr Gly Asn Gly Lys Ser
   130 135 140
   Glu Leu Ala Lys Gly Ile Lys Asp Gly Lys Lys Arg Tyr Tyr Asn Phe
   145 150 155 160
   Phe Gly Ile Gly Ala Phe Asp Ser Ser Ala Val Arg Ser Gly Lys Ser
   165 170 175
   Tyr Ala Glu Lys Glu Gln Trp Thr Ser Pro Asp Lys Ala Ile Ile Gly
   180 185 190
   Gly Ala Lys Phe Ile Arg Asn Glu Tyr Phe Glu Asn Asn Gln Leu Asn
   195 200 205
   Leu Tyr Gln Met Arg Trp Asn Pro Glu Asn Pro Ala Gln His Gln Tyr
   210 215 220
   Ala Ser Asp Ile Arg Trp Ala Asp Lys Ile Ala Lys Leu Met Asp Lys
   225 230 235 240
   Ser Tyr Lys Gln Phe Gly Ile Lys Lys Asp Asp Ile Arg Gln Thr Tyr
   245 250 255
   Tyr Lys
<210> 25
   <211> 239
   <212> PRT
   <213> Staphylococcus aureus
<400> 25
   Met Asn Lys Asn Ile Val Ile Lys Ser Met Ala Ala Leu Ala Ile Leu
   1 5 10 15
   Thr Ser Val Thr Gly Ile Asn Ala Ala Val Val Glu Glu Thr Gln Gln
   20 25 30
   Ile Ala Asn Ala Glu Lys Asn Val Thr Gln Val Lys Asp Thr Asn Asn
   35 40 45
   Phe Pro Tyr Asn Gly Val Val Ser Phe Lys Asp Ala Thr Gly Phe Val
   50 55 60
   Ile Gly Lys Asn Thr Ile Ile Thr Asn Lys His Val Ser Lys Asp Tyr
   65 70 75 80
   Lys Val Gly Asp Arg Ile Thr Ala His Pro Asn Gly Asp Lys Gly Asn
   85 90 95
   Gly Gly Ile Tyr Lys Ile Lys Ser Ile Ser Asp Tyr Pro Gly Asp Glu
   100 105 110
   Asp Ile Ser Val Met Asn Ile Glu Glu Gln Ala Val Glu Arg Gly Pro
   115 120 125
   Lys Gly Phe Asn Phe Asn Glu Asn Val Gln Ala Phe Asn Phe Ala Lys
   130 135 140
   Asp Ala Lys Val Asp Asp Lys Ile Lys Val Ile Gly Tyr Pro Leu Pro
   145 150 155 160
   Ala Gln Asn Ser Phe Lys Gln Phe Glu Ser Thr Gly Thr Ile Lys Arg
   165 170 175
   Ile Lys Asp Asn Ile Leu Asn Phe Asp Ala Tyr Ile Glu Pro Gly Asn
   180 185 190
   Ser Gly Ser Pro Val Leu Asn Ser Asn Asn Glu Val Ile Gly Val Val
   195 200 205
   Tyr Gly Gly Ile Gly Lys Ile Gly Ser Glu Tyr Asn Gly Ala Val Tyr
   210 215 220
   Phe Thr Pro Gln Ile Lys Asp Phe Ile Gln Lys His Ile Glu Gln
   225 230 235
<210> 26
   <211> 239
   <212> PRT
   <213> Staphylococcus aureus
<400> 26
   Met Asn Lys Asn Ile Ile Ile Lys Ser Ile Ala Ala Leu Thr Ile Leu
   1               5                   10                  15
   Thr Ser Val Thr Gly Val Gly Thr Thr Met Val Glu Gly Ile Gln Gln
               20                  25                  30
   Thr Ala Lys Ala Glu Asn Thr Val Lys Gln Ile Thr Asn Thr Asn Val
           35                  40                  45
   Ala Pro Tyr Ser Gly Val Thr Trp Met Gly Ala Gly Thr Gly Phe Val
       50                  55                  60
   Val Gly Asn His Thr Ile Ile Thr Asn Lys His Val Thr Tyr His Met
   65                  70                  75                  80
   Lys Val Gly Asp Glu Ile Lys Ala His Pro Asn Gly Phe Tyr Asn Asn
                   85                  90                  95
   Gly Gly Gly Leu Tyr Lys Val Thr Lys Ile Val Asp Tyr Pro Gly Lys
               100                 105                 110
   Glu Asp Ile Ala Val Val Gln Val Glu Glu Lys Ser Thr Gln Pro Lys
           115                 120                 125
   Gly Arg Lys Phe Lys Asp Phe Thr Ser Lys Phe Asn Ile Ala Ser Glu
       130                 135                 140
   Ala Lys Glu Asn Glu Pro Ile Ser Val Ile Gly Tyr Pro Asn Pro Asn
   145                 150                 155                 160
   Gly Asn Lys Leu Gln Met Tyr Glu Ser Thr Gly Lys Val Leu Ser Val
                   165                 170                 175
   Asn Gly Asn Ile Val Ser Ser Asp Ala Ile Ile Gln Pro Gly Ser Ser
               180                 185                 190
   Gly Ser Pro Ile Leu Asn Ser Lys His Glu Ala Ile Gly Val Ile Tyr
           195                 200                 205
   Ala Gly Asn Lys Pro Ser Gly Glu Ser Thr Arg Gly Phe Ala Val Tyr
       210                 215                 220
   Phe Ser Pro Glu Ile Lys Lys Phe Ile Ala Asp Asn Leu Asp Lys
   225                 230                 235
<210> 27
   <211> 189
   <212> PRT
   <213> Staphylococcus aureus
<400> 27
   Met Ser Leu Ile Asn Lys Glu Ile Leu Pro Phe Thr Ala Gln Ala Phe
   1               5                   10                  15
   Asp Pro Lys Lys Asp Gln Phe Lys Glu Val Thr Gln Glu Asp Leu Lys
               20                  25                  30
   Gly Ser Trp Ser Val Val Cys Phe Tyr Pro Ala Asp Phe Ser Phe Val
           35                  40                  45
   Cys Pro Thr Glu Leu Glu Asp Leu Gln Asn Gln Tyr Glu Glu Leu Gln
       50                  55                  60
   Lys Leu Gly Val Asn Val Phe Ser Val Ser Thr Asp Thr His Phe Val
   65                  70                  75                  80
   His Lys Ala Trp His Asp His Ser Asp Ala Ile Ser Lys Ile Thr Tyr
                   85                  90                  95
   Thr Met Ile Gly Asp Pro Ser Gln Thr Ile Thr Arg Asn Phe Asp Val
               100                 105                 110
   Leu Asp Glu Ala Thr Gly Leu Ala Gln Arg Gly Thr Phe Ile Ile Asp
           115                 120                 125
   Pro Asp Gly Val Val Gln Ala Ser Glu Ile Asn Ala Asp Gly Ile Gly
       130                 135                 140
   Arg Asp Ala Ser Thr Leu Ala His Lys Ile Lys Ala Ala Gln Tyr Val
   145                 150                 155                 160
   Arg Lys Asn Pro Gly Glu Val Cys Pro Ala Lys Trp Glu Glu Gly Ala
                   165                 170                 175
   Lys Thr Leu Gln Pro Gly Leu Asp Leu Val Gly Lys Ile
               180                 185
<210> 28
   <211> 190
   <212> PRT
   <213> Staphylococcus aureus
<400> 28
   Met Lys Leu Lys Ser Leu Ala Val Leu Ser Met Ser Ala Val Val Leu
   1               5                   10                  15
   Thr Ala Cys Gly Asn Asp Thr Pro Lys Asp Glu Thr Lys Ser Thr Glu
               20                  25                  30
   Ser Asn Thr Asn Gln Asp Thr Asn Thr Thr Lys Asp Val Ile Ala Leu
           35                  40                  45
   Lys Asp Val Lys Thr Ser Pro Glu Asp Ala Val Lys Lys Ala Glu Glu
       50                  55                  60
   Thr Tyr Lys Gly Gln Lys Leu Lys Gly Ile Ser Phe Glu Asn Ser Asn
   65                  70                  75                  80
   Gly Glu Trp Ala Tyr Lys Val Thr Gln Gln Lys Ser Gly Glu Glu Ser
                   85                  90                  95
   Glu Val Leu Val Ala Asp Lys Asn Lys Lys Val Ile Asn Lys Lys Thr
               100                 105                 110
   Glu Lys Glu Asp Thr Val Asn Glu Asn Asp Asn Phe Lys Tyr Ser Asp
           115                 120                 125
   Ala Ile Asp Tyr Lys Lys Ala Ile Lys Glu Gly Gln Lys Glu Phe Asp
       130                 135                 140
   Gly Asp Ile Lys Glu Trp Ser Leu Glu Lys Asp Asp Gly Lys Leu Val
   145                 150                 155                 160
   Tyr Asn Ile Asp Leu Lys Lys Gly Asn Lys Lys Gln Glu Val Thr Val
                   165                 170                 175
   Asp Ala Lys Asn Gly Lys Val Leu Lys Ser Glu Gln Asp His
               180                 185                 190
<210> 29
   <211> 227
   <212> PRT
   <213> Staphylococcus aureus
<400> 29
   Met Lys Leu Lys Asn Ile Ala Lys Ala Ser Leu Ala Leu Gly Ile Leu
   1               5                   10                  15
   Thr Thr Gly Met Ile Thr Thr Thr Ala Gln Pro Val Lys Ala Ser Thr
               20                  25                  30
   Leu Glu Val Arg Ser Gln Ala Thr Gln Asp Leu Ser Glu Tyr Tyr Lys
           35                  40                  45
   Gly Arg Gly Phe Glu Leu Thr Asn Val Thr Gly Tyr Lys Tyr Gly Asn
       50                  55                  60
   Lys Val Thr Phe Ile Asp Asn Ser Gln Gln Ile Asp Val Thr Leu Thr
   65                  70                  75                  80
   Gly Asn Glu Lys Leu Thr Val Lys Asp Asp Asp Glu Val Ser Asn Val
                   85                  90                  95
   Asp Val Phe Val Val Arg Glu Gly Ser Asp Lys Ser Ala Ile Thr Thr
               100                 105                 110
   Ser Ile Gly Gly Ile Thr Lys Thr Asn Gly Thr Gln His Lys Asp Thr
           115                 120                 125
   Val Gln Asn Val Asn Leu Ser Val Ser Lys Ser Thr Gly Gln His Thr
       130                 135                 140
   Thr Ser Val Thr Ser Glu Tyr Tyr Ser Ile Tyr Lys Glu Glu Ile Ser
   145                 150                 155                 160
   Leu Lys Glu Leu Asp Phe Lys Leu Arg Lys His Leu Ile Asp Lys His
                   165                 170                 175
   Asp Leu Tyr Lys Thr Glu Pro Lys Asp Ser Lys Ile Arg Ile Thr Met
               180                 185                 190
   Lys Asn Gly Gly Tyr Tyr Thr Phe Glu Leu Asn Lys Lys Leu Gln Pro
           195                 200                 205
   His Arg Met Gly Asp Thr Ile Asp Ser Arg Asn Ile Glu Lys Ile Glu
       210                 215                 220
   Val Asn Leu
   225
<210> 30
   <211> 226
   <212> PRT
   <213> Staphylococcus aureus
<400> 30
   Met Lys Phe Lys Ala Ile Ala Lys Ala Ser Leu Ala Leu Gly Met Leu
   1               5                   10                  15
   Ala Thr Gly Val Ile Thr Ser Asn Val Gln Ser Val Gln Ala Lys Thr
               20                  25                  30
   Glu Val Lys Gln Gln Ser Glu Ser Glu Leu Lys His Tyr Tyr Asn Lys
           35                  40                  45
   Pro Val Leu Glu Arg Lys Asn Val Thr Gly Tyr Lys Tyr Thr Glu Lys
       50                  55                  60
   Gly Lys Asp Tyr Ile Asp Val Ile Val Asp Asn Gln Tyr Ser Gln Ile
   65                  70                  75                  80
   Ser Leu Val Gly Ser Asp Lys Asp Lys Phe Lys Asp Gly Asp Asn Ser
                   85                  90                  95
   Asn Ile Asp Val Phe Ile Leu Arg Glu Gly Asp Ser Arg Gln Ala Thr
               100                 105                 110
   Asn Tyr Ser Ile Gly Gly Val Thr Lys Thr Asn Ser Gln Pro Phe Ile
           115                 120                 125
   Asp Tyr Ile His Thr Pro Ile Leu Glu Ile Lys Lys Gly Lys Glu Glu
       130                 135                 140
   Pro Gln Ser Ser Leu Tyr Gln Ile Tyr Lys Glu Asp Ile Ser Leu Lys
   145                 150                 155                 160
   Glu Leu Asp Tyr Arg Leu Arg Glu Arg Ala Ile Lys Gln His Gly Leu
                   165                 170                 175
   Tyr Ser Asn Gly Leu Lys Gln Gly Gln Ile Thr Ile Thr Met Lys Asp
               180                 185                 190
   Gly Lys Ser His Thr Ile Asp Leu Ser Gln Lys Leu Glu Lys Glu Arg
           195                 200                 205
   Met Gly Asp Ser Ile Asp Gly Arg Gln Ile Gln Lys Ile Leu Val Glu
       210                 215                 220
   Met Lys
   225
<210> 31
   <211> 170
   <212> PRT
   <213> Staphylococcus aureus
<400> 31
   Met Thr Leu Phe Leu Leu Glu Ala Asn Asn Leu Asp Phe Ala Ser Thr
   1               5                   10                  15
   Lys Glu Glu Leu Glu Ala Lys Ala Ala Ser Leu Ser Thr Lys Thr Ile
               20                  25                  30
   Pro Thr Leu Ile Glu Val Gln Ala Thr Glu Asn Leu Thr His Gly Tyr
           35                  40                  45
   Phe Ile Val Glu Ala Asn Asp Glu Ala Glu Ala Lys Gln Phe Leu Thr
       50                  55                  60
   Glu Ala Asp Ile Ser Ile Gln Leu Val Lys Glu Val Arg Leu Val Gly
   65                  70                  75                  80
   Lys Asp Leu Asp Glu Val Lys Asn Gly Asp Ala His Val Asp Tyr Leu
                   85                  90                  95
   Val Thr Trp Asn Ile Pro Glu Gly Ile Thr Met Asp Gln Tyr Leu Ala
               100                 105                 110
   Arg Lys Lys Lys Asn Ser Val His Tyr Glu Glu Val Pro Glu Val Glu
           115                 120                 125
   Phe Lys Arg Thr Tyr Val Cys Glu Asp Met Ser Lys Cys Ile Cys Leu
       130                 135                 140
   Tyr Asn Ala Pro Asp Glu Glu Ala Val Arg Arg Ala Arg Lys Ala Val
   145                 150                 155                 160
   Asp Thr Pro Ile Asp Gly Ile Glu Lys Leu
                   165                 170
<210> 32
   <211> 168
   <212> PRT
   <213> Staphylococcus aureus
<400> 32
   Met Lys Lys Leu Leu Thr Ala Ser Ile Ile Ala Cys Ser Val Val Met
   1               5                   10                  15
   Gly Val Gly Leu Val Asn Thr Ser Ala Glu Ala Ala Ser Gly Asn Ser
               20                  25                  30
   Ile Asp Thr Val Lys Gln Leu Ile Lys Gly Asp Gln Ser Leu Glu Asn
           35                  40                  45
   Val Lys Ile Gly Glu Ser Ile Lys Asp Val Leu Thr Lys Tyr Lys Asn
       50                  55                  60
   Pro Met Tyr Ser Tyr Asn Glu Asp Gly Thr Glu His Tyr Tyr Glu Phe
   65                  70                  75                  80
   His Thr Lys Lys Gly Met Leu Leu Val Thr Thr Asp Gly Lys Lys Asn
                   85                  90                  95
   Asn Gly Lys Val Thr His Ile Ser Met Met Tyr Asn Asp Ala Asn Gly
               100                 105                 110
   Pro Thr Tyr Gln Ala Val Lys Asn Tyr Val Gly Lys Ala Val Thr His
           115                 120                 125
   Thr Glu Tyr Ser Lys Val Ala Gly Asn Phe Gly Tyr Ile Glu Lys Gly
       130                 135                 140
   Lys Thr Thr Tyr Gln Phe Ala Ser Ala Pro Lys Asp Lys Asn Ile Lys
   145                 150                 155                 160
   Leu Tyr Arg Ile Asp Leu Glu Lys
                   165
<210> 33
   <211> 228
   <212> PRT
   <213> Staphylococcus aureus
<400> 33
   Met Thr Glu Tyr Leu Leu Ser Ala Gly Ile Cys Met Ala Ile Val Ser
   1               5                   10                  15
   Ile Leu Leu Ile Gly Met Ala Ile Ser Asn Val Ser Lys Gly Gln Tyr
               20                  25                  30
   Ala Lys Arg Phe Phe Phe Phe Ala Thr Ser Cys Leu Val Leu Thr Leu
           35                  40                  45
   Val Val Val Ser Ser Leu Ser Ser Ser Ala Asn Ala Ser Gln Thr Asp
       50                  55                  60
   Asn Gly Val Asn Arg Ser Gly Ser Glu Asp Pro Thr Val Tyr Ser Ala
   65                  70                  75                  80
   Thr Ser Thr Lys Lys Leu His Lys Glu Pro Ala Thr Leu Ile Lys Ala
                   85                  90                  95
   Ile Asp Gly Asp Thr Val Lys Leu Met Tyr Lys Gly Gln Pro Met Thr
               100                 105                 110
   Phe Arg Leu Leu Leu Val Asp Thr Pro Glu Thr Lys His Pro Lys Lys
           115                 120                 125
   Gly Val Glu Lys Tyr Gly Pro Glu Ala Ser Ala Phe Thr Lys Lys Met
       130                 135                 140
   Val Glu Asn Ala Asn Lys Ile Glu Val Glu Phe Asp Lys Gly Gln Arg
   145                 150                 155                 160
   Thr Asp Lys Tyr Gly Arg Gly Leu Ala Tyr Ile Tyr Ala Asp Gly Lys
                   165                 170                 175
   Met Val Asn Glu Ala Leu Val Arg Gln Gly Leu Ala Lys Val Ala Tyr
               180                 185                 190
   Val Tyr Lys Pro Asn Asn Thr His Glu Gln Leu Leu Arg Lys Ser Glu
           195                 200                 205
   Ala Gln Ala Lys Lys Glu Lys Leu Asn Ile Trp Ser Glu Asp Asn Ala
       210                 215                 220
   Asp Ser Gly Gln
   225
<210> 34
   <211> 169
   <212> PRT
   <213> Staphylococcus aureus
<400> 34
<210> 35
   <211> 166
   <212> PRT
   <213> Staphylococcus aureus
<400> 35
   Met Ile Thr Tyr Lys Asn Ile Leu Ile Ala Val Asp Gly Ser His Glu
   1               5                   10                  15
   Ala Glu Trp Ala Phe Asn Arg Ala Val Gly Val Ala Lys Arg Asn Asp
               20                  25                  30
   Ala Lys Leu Thr Ile Val Asn Val Ile Asp Ser Arg Thr Tyr Ser Ser
           35                  40                  45
   Tyr Glu Val Tyr Asp Ala Gln Phe Thr Glu Lys Ser Lys His Phe Ala
       50                  55                  60
   Glu Glu Leu Leu Asn Gly Tyr Lys Glu Val Ala Thr Asn Ala Gly Val
   65                  70                  75                  80
   Lys Asp Val Glu Thr Arg Leu Glu Phe Gly Ser Pro Lys Ser Ile Ile
                   85                  90                  95
   Pro Lys Lys Leu Ala His Glu Ile Asn Ala Asp Leu Ile Met Ser Gly
               100                 105                 110
   Thr Ser Gly Leu Asn Ala Val Glu Arg Phe Ile Val Gly Ser Val Ser
           115                 120                 125
   Glu Ser Ile Val Arg His Ala Pro Cys Asp Val Leu Val Val Arg Thr
       130                 135                 140
   Glu Glu Leu Pro Ala Asp Phe Gln Pro Gln Val Ala Thr Thr Gln Leu
   145                 150                 155                 160
   Arg Glu Lys Tyr Gln Asn
                   165
<210> 36
   <211> 141
   <212> PRT
   <213> Staphylococcus aureus
<400> 36
   Met Lys Leu Lys Ser Phe Val Thr Ala Thr Leu Ala Leu Gly Leu Leu
   1               5                   10                  15
   Ser Thr Val Gly Ala Ala Leu Pro Ser His Glu Ala Ser Ala Asp Ser
                   20                  25                  30
   Asn Asn Gly Tyr Lys Glu Leu Thr Met Asp Gly Lys His Thr Val Pro
           35                  40                  45
   Tyr Thr Ile Ser Val Asp Gly Ile Thr Ala Leu His Arg Thr Tyr Phe
       50                  55                  60
   Val Phe Pro Glu Asn Lys Lys Val Leu Tyr Gln Glu Ile Asp Ser Lys
   65                  70                  75                  80
   Val Lys Asn Glu Leu Ala Ser Gln Arg Gly Val Thr Thr Glu Lys Ile
                   85                  90                  95
   Asn Asn Ala Gln Thr Ala Thr Tyr Thr Leu Thr Leu Asn Asp Gly Asn
               100                 105                 110
   Lys Lys Val Val Asn Leu Lys Lys Asn Asp Asp Ala Lys Asn Ser Ile
           115                 120                 125
   Asp Pro Ser Thr Ile Lys Gln Ile Gln Ile Val Val Lys
       130                 135                 140
<210> 37
   <211> 173
   <212> PRT
   <213> Staphylococcus aureus
<400> 37
   Met Lys Arg Lys Val Leu Val Leu Thr Met Gly Val Ile Cys Ala Thr
   1 5 10 15
   Gln Leu Trp His Ser Asn His Ala Asn Ala Leu Val Thr Glu Ser Val
   20 25 30
   Glu Thr Asn Phe Val Val Lys Asp Ser Asp Asn Lys Asn Ile Leu Gln
   35 40 45
   Thr His Thr Glu Ile Thr Thr Glu Glu Lys Phe Ser Ile Val Glu Lys
   50 55 60
   Ser Gln Leu Asn Lys Leu Lys Ala Leu Ser Asn Asp Asn Tyr Ile Glu
   65 70 75 80
   Tyr Asp Leu His Thr Asn Gln Thr Gly Ile Lys Lys Gly Trp Leu Tyr
   85 90 95
   Gly Tyr Ser Glu Ile Asp Ser Ser His Phe Thr Asp Arg Asp Lys Arg
   100 105 110
   Val Ile Arg Arg Asp His Val Lys Glu Ala Gln Ser Leu Ile Asn Asp
   115 120 125
   Tyr Lys Asp Thr Gln Ser Tyr Glu Asp Leu Ala Lys Ala Thr Ala Lys
   130 135 140
   Val Ser Thr Leu Ser Gln Ser His Gln Asn Tyr Leu Asn Lys Gln Ile
   145 150 155 160
   Asp Lys Val Asn Asn Lys Ile Glu Lys Thr Glu Lys Arg
   165 170
<210> 38
   <211> 175
   <212> PRT
   <213> Staphylococcus aureus
<400> 38
   Met Asn Lys Thr Ser Lys Val Cys Val Ala Ala Thr Leu Ala Leu Gly
   1               5                   10                  15
   Thr Leu Ile Gly Val Thr Val Val Glu Asn Ser Ala Pro Thr Ser Lys
               20                  25                  30
   Gln Ala Gln Ala Ala Ile Thr Pro Tyr Tyr Thr Tyr Asn Gly Tyr Ile
           35                  40                  45
   Gly Asn Asn Ala Asn Phe Ile Leu Asp Lys Asn Phe Ile Asn Ala Ile
       50                  55                  60
   Lys Tyr Asp Asn Val Lys Phe Asn Gly Ile Lys Leu Ala Lys Thr Asn
   65                  70                  75                  80
   Thr Ile Lys Lys Val Glu Lys Tyr Asp Gln Thr Phe Lys Gly Val Ser
                   85                  90                  95
   Ala Lys Gly Asn Glu Ala Ser Gln Leu Gln Phe Val Val Lys Asn Asn
               100                 105                 110
   Ile Ser Leu Lys Asp Ile Gln Lys Ala Tyr Gly Lys Asp Leu Lys Lys
           115                 120                 125
   Glu Asn Gly Lys Thr Lys Glu Ala Asp Ser Gly Ile Phe Tyr Tyr Gln
       130                 135                 140
   Asn Ala Lys Lys Thr Leu Gly Ile Trp Phe Val Val Asp His Asn Arg
   145                 150                 155                 160
   Val Val Glu Val Thr Val Gly His Thr Pro Tyr Lys Thr Ser Lys
               165                 170                 175
<210> 39
   <211> 163
   <212> PRT
   <213> Staphylococcus aureus
<400> 39
   Met Leu Lys Arg Ser Leu Leu Phe Leu Thr Val Leu Leu Leu Leu Phe
   1               5                   10                  15
   Ser Phe Ser Ser Ile Thr Asn Glu Val Ser Ala Ser Ser Ser Phe Asp
               20                  25                  30
   Lys Gly Lys Tyr Lys Lys Gly Asp Asp Ala Ser Tyr Phe Glu Pro Thr
           35                  40                  45
   Gly Pro Tyr Leu Met Val Asn Val Thr Gly Val Asp Gly Lys Gly Asn
       50                  55                  60
   Glu Leu Leu Ser Pro His Tyr Val Glu Phe Pro Ile Lys Pro Gly Thr
   65                  70                  75                  80
   Thr Leu Thr Lys Glu Lys Ile Glu Tyr Tyr Val Glu Trp Ala Leu Asp
                   85                  90                  95
   Ala Thr Ala Tyr Lys Glu Phe Arg Val Val Glu Leu Asp Pro Ser Ala
               100                 105                 110
   Lys Ile Glu Val Thr Tyr Tyr Asp Lys Asn Lys Lys Lys Glu Glu Thr
           115                 120                 125
   Lys Ser Phe Pro Ile Thr Glu Lys Gly Phe Val Val Pro Asp Leu Ser
       130                 135                 140
   Glu His Ile Lys Asn Pro Gly Phe Asn Leu Ile Thr Lys Val Val Ile
   145                 150                 155                 160
   Glu Lys Lys
<210> 40
   <211> 144
   <212> PRT
   <213> Staphylococcus aureus
<400> 40
   Met Lys Leu Lys Ser Phe Ile Thr Val Thr Leu Ala Leu Gly Met Ile
   1               5                   10                  15
   Ala Thr Thr Gly Ala Thr Val Thr Ser Asn Glu Val Ser Ala Ala Glu
               20                  25                  30
   Lys Asp Lys Leu Pro Ala Thr Gln Lys Ala Lys Glu Met Gln Asn Val
           35                  40                  45
   Pro Tyr Thr Ile Ala Val Asp Gly Ile Met Ala Phe Asn Gln Ser Tyr
       50                  55                  60
   Leu Asn Leu Pro Lys Asp Ser Gln Leu Ser Tyr Leu Asp Leu Gly Asn
   65                  70                  75                  80
   Lys Val Lys Ala Leu Leu Tyr Asp Glu Arg Gly Val Thr Pro Glu Lys
                   85                  90                  95
   Ile Arg Asn Ala Lys Ser Ala Val Tyr Thr Ile Thr Trp Lys Asp Gly
               100                 105                 110
   Ser Lys Lys Glu Val Asp Leu Lys Lys Asp Ser Tyr Thr Ala Asn Leu
           115                 120                 125
   Phe Asp Ser Asn Ser Ile Lys Gln Ile Asp Ile Asn Val Lys Thr Lys
       130                 135                 140
<210> 41
   <211> 116
   <212> PRT
   <213> Staphylococcus aureus
<400> 41
   Met Lys Ile Arg Lys Ser Ile Leu Ala Gly Thr Leu Ala Ile Val Leu
   1               5                   10                  15
   Ala Ser Pro Leu Val Thr Asn Leu Asp Lys Asn Glu Ala Gln Ala Ser
               20                  25                  30
   Thr Ser Leu Pro Thr Ser Asn Glu Tyr Gln Asn Glu Lys Leu Ala Asn
           35                  40                  45
   Glu Leu Lys Ser Leu Leu Asp Glu Leu Asn Val Asn Glu Leu Ala Thr
       50                  55                  60
   Gly Ser Leu Asn Thr Tyr Tyr Lys Arg Thr Ile Lys Ile Ser Gly Gln
   65                  70                  75                  80
   Lys Ala Met Tyr Ala Leu Lys Ser Lys Asp Phe Lys Lys Met Ser Glu
                   85                  90                  95
   Ala Lys Tyr Gln Leu Gln Lys Ile Tyr Asn Glu Ile Asp Glu Ala Leu
               100                 105                 110
   Lys Ser Lys Tyr
           115
<210> 42
   <211> 241
   <212> PRT
   <213> Staphylococcus aureus
<400> 42
   Met Ser Asn Glu Ile Leu Ile Val Asp Asp Glu Asp Arg Ile Arg Arg
   1               5                   10                  15
   Leu Leu Lys Met Tyr Leu Glu Arg Glu Ser Phe Glu Ile His Glu Ala
               20                  25                  30
   Ser Asn Gly Gln Glu Ala Tyr Glu Leu Ala Met Glu Asn Asn Tyr Ala
           35                  40                  45
   Cys Ile Leu Leu Asp Leu Met Leu Pro Glu Met Asp Gly Ile Gln Val
       50                  55                  60
   Ala Thr Lys Leu Arg Glu His Lys Gln Thr Pro Ile Ile Met Leu Thr
   65                  70                  75                  80
   Ala Lys Gly Glu Glu Thr Asn Arg Val Glu Gly Phe Glu Ser Gly Ala
                   85                  90                  95
   Asp Asp Tyr Ile Val Lys Pro Phe Ser Pro Arg Glu Val Val Leu Arg
               100                 105                 110
   Val Lys Ala Leu Leu Arg Arg Thr Gln Ser Thr Thr Val Glu Gln Ser
           115                 120                 125
   Glu Pro His Ala Arg Asp Val Ile Glu Phe Lys His Leu Glu Ile Asp
       130                 135                 140
   Asn Asp Ala His Arg Val Leu Ala Asp Asn Gln Glu Val Asn Leu Thr
   145                 150                 155                 160
   Pro Lys Glu Tyr Glu Leu Leu Ile Tyr Leu Ala Lys Thr Pro Asn Lys
                   165                 170                 175
   Val Phe Asp Arg Glu Gln Leu Leu Lys Glu Val Trp His Tyr Glu Phe
               180                 185                 190
   Tyr Gly Asp Leu Arg Thr Val Asp Thr His Val Lys Arg Leu Arg Glu
           195                 200                 205
   Lys Leu Asn Arg Val Ser Ser Glu Ala Ala His Met Ile Gln Thr Val
       210                 215                 220
   Trp Gly Val Gly Tyr Lys Phe Glu Val Lys Ser Asn Asp Glu Pro Ala
   225                 230                 235                 240
   Lys

## Description

### Domaine de l'invention

La présente invention concerne une méthode de diagnostic des infections à staphylocoques.

### Arrière plan technique

Plus de 10 millions d'individus dans le monde sont porteurs de prothèses ostéo-articulaires (hanche, genou, épaule) et ce nombre croît régulièrement du fait du vieillissement de la population et des problèmes de surpoids.

Les infections sur prothèse articulaire (IPOA) sont une des principales complications liées à la pose d'une prothèse. Elles sont associées à une très forte morbidité, car elles nécessitent une ou plusieurs ré-interventions, une longue antibiothérapie, et entraînent un handicap fonctionnel important, souvent prolongé. Les IPOA sont dans 50 à 75% des cas causées par des bactéries appartenant au genre *Staphylococcus.* Une des principales espèces impliquées est *S*. *aureus.*

Le diagnostic d'IPOA résulte d'un faisceau d'arguments cliniques, paradliniques et microbiologiques, qui dans certains cas ne permettent pas d'aboutir à un diagnostic certain. La culture de prélèvements reste la technique de préférence nécessitant un geste invasif et sujet à la contamination bactérienne. Il n'existe actuellement pas d'outil sérologique pour le diagnostic et le suivi d'IPOA.

De nombreuses séquences peptidiques et nucléotidiques issues de S. *aureus sont* décrites dans l'art antérieur. Ainsi, POCSFALVI ET AL Proteomics. 2008 Jun; 8(12):2462-76 a identifié à l'aide d'une approche protéomique, 119 exoprotéines de *S*. *aureus.* WO 02/094868 décrit 5642 séquences nucléiques ou peptidique issues de S. *aureus* dans le diagnostic d'une infection par *S*. *aureus* chez un individu. B. CHRISTENSSON et al -Acta Pathol Microbiol Immunol Scand B. 1983 Oct; 91(5):351-6 montre que par rapport à des individus sains, les patients souffrant d'une infection à *S. aureus* ont des taux beaucoup plus élevés d'anticorps dirigés contre la toxine alpha-hémolysine issue de *S. aureus,* qu'un groupe de patients ayant développé des septicémies qui ne sont pas dues à *S*. *aureus* qui eux, ont des niveaux d'anticorps normaux.

### Résumé de l'invention

L'objectif de l'étude était d'identifier des antigènes pertinents pour le sérodiagnostic d'IPOA à *S*. *aureus.* Les inventeurs ont procédé à l'analyse de la réponse humorale anti-staphylococcique de patients atteints d'IPOA à *S*. *aureus.* L'étude a été réalisée par la technique d'immunoprotéome à partir des protéines bactériennes sécrétées dans le milieu extracellulaire. Deux souches de *S*. *aureus* résistante (SARM) et sensible (SASM) à la méthicilline ont été étudiées en parallèle.

Les inventeurs ont ainsi notamment montré que les protéines de séquences SEQ ID NO: 1, SEQ ID NO: 6, SEQ ID NO: 9, SEQ ID NO: 11, SEQ ID NO: 18, SEQ 10 NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41, et SEQ ID NO: 42 sont utiles pour le diagnostic sérologique des infections à staphylocoques, notamment dans le cadre des infections sur prothèses ostéo-articulaires.

La présente demande décrit donc une méthode *in vitro* pour déterminer si un individu est infecté par une bactérie du genre *Staphylococcus,* comprenant la détection d'anticorps dirigés contre au moins une protéine comprenant ou constituée d'une séquence choisie parmi SEQ 10 NO: 1, SEQ ID NO: 6, SEQ ID NO: 9, SEQ ID NO: 11, SEQ ID NO: 18, SEQ ID NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41 et SEQ ID NO: 42, dans un échantillon biologique de l'individu.

L'invention est telle que définie dans les revendications.

### Description détaillée de l'invention

### Définitions

Comme on l'entend ici l'expression « *bactérie du genre Staphylococcus »* se réfère à toutes les espèces ou sous-espèces comprises dans ce genre. On préfère toutefois que la bactérie du genre *Staphylococcus soit Staphylococcus aureus, Staphylococcus epidermidis, Staphylococcus capitis, Staphylococcus lugdunensis, Staphylococcus caprae, Staphylococcus warnerii ou Staphylococcus hominis.*

Comme on l'entend ici l'expression "infecté" ou "infection" concerne des individus porteurs d'une bactérie du genre *Staphylococcus* telle que définie ci-dessus. Les individus infectés peuvent présenter un ou plusieurs sites de multiplication des bactéries du genre *Staphylococcus.*

Les infections par des bactéries du genre *Staphylococcus* peuvent être la conséquence d'une intervention chirurgicale pour la pose d'un matériel étranger. Ainsi, comme on l'entend ici, l'individu a de préférence subi une intervention chirurgicale, et, en particulier, a subi l'implantation d'une prothèse, telle qu'une prothèse articulaire, notamment sélectionnée dans le groupe constitué d'une prothèse du genou, une prothèse de hanche et une prothèse d'épaule.

L'infection diagnostiquée dans le cadre de la présente invention, et pour laquelle on souhaite déterminer si elle est due à la présence d'une bactérie du genre *Staphylococcus,* peut par exemple être choisie parmi :
- une infection sur prothèse (en particulier articulaire) ;
- une infection ostéo-articulaire ;
- une infection post-opératoire (en particulier lors de la pose d'un matériel étranger tel qu'une prothèse) ;
- une myosite aiguë ;
- une otite ;
- une sinusite ;
- une infection cutanée suppurative telle qu'un furoncle, un anthrax, un panaris, une folliculite, un sycosis (par exemple en association avec un trichophyton), une cellulite, un érysipèle, un pemphigus néonatal, un impétigo (par exemple en association avec des streptocoques) ou une mammite (en particulier chez la vache) ;
- une infection de viscères telle qu'une pneumonie (surtout en tant que complication de la grippe), une endocardite (en particulier chez les patients porteurs de prothèses cardiaques), une infection urinaire, une phlébite, une entérite ou une méningite ;
- une infection des os (en particulier l'ostéomyélite) ; et
- une septicémie (en particulier chez des individus immunodéprimés).

Comme on l'entend ici, l'expression « échantillon biologique » inclut à la fois l'échantillon tel que prélevé et l'échantillon ayant été soumis à divers traitement, notamment pour le rendre apte à son utilisation dans les procédés et méthodes selon l'invention. L'« échantillon biologique » selon l'invention peut être de tout type susceptible de contenir des anticorps dirigés contre une bactérie du genre *Staphylococcus* ou de contenir des antigène issus d'une bactérie du genre *Staphylococcus,* on préfère toutefois que l'échantillon biologique soit sélectionné dans le groupe constitué d'un échantillon de sang, d'un échantillon de sérum, d'un échantillon de plasma, d'un échantillon de tissu lymphoïde associé à une muqueuse (MALT), d'un échantillon de liquide céphalo-rachidien, d'un échantillon de liquide articulaire, d'un échantillon de liquide pleural, d'un échantillon de salive, et d'un échantillon d'urine.

Comme on l'entend ici, l'expression « déterminer si un individu est infecté par une bactérie du genre *Staphylococcus* » comprend la pose d'un diagnostic ou le diagnostic d'une infection par une bactérie du genre *Staphylococcus* telle que définie ci-dessus chez individu. Elle comprend également le suivi d'individus ayant subi une intervention chirurgicale, notamment en vue d'implanter, de nettoyer ou de remplacer une prothèse. Elle comprend en outre le suivi d'une infection par une bactérie du genre *Staphylococcus* telle que définie ci-dessus, notamment dans le cadre d'un traitement thérapeutique visant à lutter contre l'infection.

Fans le cadre de la présente demande, « l'individu » est de préférence un être humain. Toutefois, il peut également s'agir d'un autre mammifère, tel que par exemple le singe, le cochon d'inde, le cobaye, le lapin, le rat, la souris, la vache, le chien, le chat ou le cheval.

Comme on l'entend ici, les expressions « protéine constituée d'une séquence » ou « protéine représentée par une séquence » sont synonymes.

Par « ligand de capture », on entend ici un composé capable de se lier, de préférence spécifiquement, à l'antigène ou à l'anticorps que l'on souhaite détecter chez un individu.

Par « ligand de détection », on entend ici un composé capable de se lier, de préférence spécifiquement, au ligand de capture et/ou au complexe formé entre le ligand de capture et l'antigène ou l'anticorps que l'on souhaite détecter et/ou à l'antigène ou l'anticorps que l'on souhaite détecter.

Le terme « spécifique » ou « spécifiauement », lorsqu'il se réfère à la liaison d'un ligand avec une première cible, signifie que le ligand peut se lier à la première cible sans pour autant se lier à une deuxième cible qui ne ressemble pas structurellement à la première cible.

Comme on l'entend ici, un "anticorps"; lorsqu'il s'agit d'un ligand de capture ou d'un ligand de détection, peut appartenir à n'importe quelle espèce, et est notamment un anticorps humain, de souris, de rat, de chèvre ou de camelidae. L'anticorps peut également être un anticorps chimérique, c'est-à-dire un anticorps constitué de parties provenant de différentes espèces. Les anticorps chimériques préférés sont dits humanisés, dans lesquels les parties constantes (notamment CH et CL) sont d'origine humaine et les parties variables (VH et VL) ou les CDR des parties variables sont d'une autre espèce, la souris par exemple. L'anticorps peut être produit par toute méthode connue de l'homme du métier, par exemple par immunisation d'un animal ou par de méthodes recombinantes ou chimiques. En outre, le terme « anticorps » comme on l'entend ici, comprend également les fragments d'anticorps qui comprennent au moins un des paratopes de l'anticorps, à savoir notamment les fragments Fab, F(ab')2, et scFv. L'anticorps peut être un anticorps polyclonal, notamment un anticorps polyclonal monospécifique, ou un anticorps monoclonal.

Les "aptamères" sont bien connus de l'homme du métier. Ils peuvent être de nature nucléotidique comme décrit notamment par Ellington et al. (1990) Nature 346:818-22 et Bock et al. (1992) Nature 355:564-6, ou de nature peptidique, comme décrits, notamment, par Hoppe-Seyler et al. (2000) J. Mol Med. 78:426-30.

La méthode dite du "phage display" est une méthode de sélection de ligands polypeptidiques exprimés sur la capside d'un bactériophage et codés par une séquence nucléotidique insérée dans le gène codant la capside. Cette méthode est bien connue de l'homme du métier et est notamment décrite par Scott & Smith (1990) Science 249:386-390, et Marks et al. (1991) J. Mol. Biol. 222:581-597. De préférence, le polypeptide obtenu par phage display est un polypeptide du type scFv (pour « single-chain variable fragment »), comme cela est notamment décrit par Winter et al. (1994) Annu. Rev. Immunol. 12:433-455.

### Polypeptides

Les inventeurs ont identifiés plusieurs nouveaux antigènes utiles pour le diagnostic sérologique des infections à staphylocoques, à savoir des antigènes de séquences SEQ ID NO: 1, SEQ ID NO: 6, SEQ ID NO: 9, SEQ ID NO: 11, SEQ ID NO: 18, SEQ ID NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41 SEQ ID NO: 42.

De ce fait, sont décrits des méthodes, utilisations et kits basés sur l'emploi ou la détection des polypeptides suivants
- une protéine comprenant ou constituée d'une séquence sélectionnée dans le groupe constitué de SEQ ID NO: 1, SEQ ID NO: 6, SEQ ID NO: 9, SEQ ID NO: 11, SEQ ID NO: 18, SEQ 6D NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41 et SEQ ID NO: 42 ; ou
- une protéine homologue comprenant ou constituée d'une séquence partageant au moins 80%, 85%, 90%. 95%, 96%, 97%, 98% ou 99% d'identité avec une séquence sélectionnée dans le groupe constitué de SEQ ID NO: 1, SEQ ID NO: 6, SEQ ID NO: 9, SEQ ID NO: 11, SEQ ID NO: 18, SEQ ID NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41 et SEQ ID NO: 42; ou
- une protéine comprenant ou constituée d'un fragment de la protéine définie en (i) ou en (ii), ledit fragment comprenant au moins 10 acides aminés consécutifs de ladite protéine définie en (i) ou en (ii);
sous réserve que la protéine définie en (ii) ou que le fragment défini en (iii) puisse être lié par au moins anticorps dirigé contre une protéine comprenant ou constituée d'une séquence sélectionnée dans le groupe constitué de SEQ ID NO: 1, SEQ ID NO: 6, SEQ ID NO: 9, SEQ ID NO: 11, SEQ ID NO: 18, SEQ ID NO: 20, SEQ ID NO: 24, SEQ ID NO: 25, SEQ ID NO: 26, SEQ ID NO: 28, SEQ ID NO: 29, SEQ ID NO: 30, SEQ ID NO: 31, SEQ ID NO: 32, SEQ ID NO: 36, SEQ ID NO: 37, SEQ ID NO: 40, SEQ ID NO: 41 et SEQ ID NO: 42.

Comme on l'entend ici, la protéine homologue définie ci-dessus ou le fragment défini ci-dessus, est tel qu'il puisse être lié par au moins un anticorps dirigé contre une protéine représentée par une séquence sélectionnée dans le groupe constitué de SEQ ID Nos. 1, 6, 9, 11, 18, 20, 24, 25, 26, 28, 29, 30, 31, 32, 36, 37, 40,41 et 42. En d'autres termes, la protéine homologue définie ci-dessus ou le fragment défini ci-dessus comprennent au moins un des épitopes d'une protéine constituée de 1, 6, 9, 11, 18, 20, 24, 25, 26, 28, 29, 30, 31, 32, 36, 37, 40, 41 et 42. En conséquence, la protéine homologue définie ci-dessus ou le fragment défini ci-dessus devraient de préférence être tels qu'ils procurent au moins 70%, plus préférablement au moins 80% et encore plus préférablement au moins 90%, de la sensibilité respectivement procurée par une protéine comprenant ou constituée de SEQ ID Nos. 1, 6, 9, 11, 18, 20, 24, 25, 26, 28, 29, 30, 31, 32, 36, 37, 40, 41 et 42, mesurée dans les mêmes conditions. Comme on l'entend ici, le terme « sensibilité » est défini comme le pourcentage d'individus infectés par une bactérie du genre *Staphylococcus* telle que définie ci-dessus, de préférence par *S*. *aureus,* dont les échantillons biologiques, notamment des échantillons de sérum, contiennent des anticorps dirigés contre une protéine comprenant ou constituée de SEQ ID Nos. 1, 6, 9, 11, 18, 20, 24, 25, 26, 28, 29, 30, 31, 32, 36, 37, 40, 41 et42 détectables.

Les polypeptides tels que définis ci-dessus peuvent être composés des 20 acides aminés naturels ou d'acides aminés que ces 20 acides aminés codés. Ils peuvent également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits de la chaîne polypeptidique et n'importe où dans la chaîne polypeptidique : dans le squelette peptidique, dans les groupes latéraux ou encore aux extrémités carboxy- ou amino-terminales. Ils peuvent être ramifié suite à une ubiquitination ou être cyclique avec ou sans ramification. Ce type de modifications peut être le résultat de processus de post-traduction naturels ou synthétiques, qui sont bien connus de l'homme du métier.

On entend, par exemple, par modifications d'un polypeptide, l'acétylation, l'acylation, l'ADP-ribosylation, l'amidation, la fixation covalence de flavine, la fixation covalente d'un hème, la fixation covalente d'un nucléotide ou d'un dérivé nucléotidique, la fixation covalente d'un lipide ou d'un dérivé lipidique, la fixation covalence d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, la palmitoylation, la glypiation, l'oxydation, le processus protéolytique, la phosphorylation, la prénylation, la racémisation, la sénéloylation, la sulfatation, l'addition d'acides aminés, telle que l'arginylation ou l'ubiquitination. (PROTEINS STRUCTURE AND MOLECULAR PROPERTIES, 2nd Ed., T.E. Creighton, W.H. Freeman and Company, New York (1993) and Wold, F., Posttranslational Protein Modifications : Perspectives and Prospects, pgs 1-12 in POSTTRANSLATIONAL COVALENT MODIFICATION (OF PROTEINS, B.C. Johnson, Ed., Academic Press, New York (1983) ; Seifter et al., Meth. Enzymol. 182 : 626-646 (1990) and Rattan et al., Protein Synthesis : Posttranslational Modifications and Aging, Ann. N.Y. Acad. Sci. 663 : 48-62 (1992)).

Par ailleurs, lorsqu'ils sont obtenus par voie recombinante, les polypeptides tels que définis ci-dessus peuvent également comprendre des séquences utiles pour la purification des protéines (des étiquettes de purification), telles que des étiquettes polyhistidine, et éventuellement une séquence permettant de cliver ces étiquettes, par exemple des sites de coupures par des protéases.

De préférence, un polypeptide comprenant une séquence sélectionnée dans le groupe constitué de SEQ ID Nos. 1, 6, 9, 11, 18, 20) 24, 25, 26, 28, 29, 30, 31, 32, 36, 37, 40, 41 et 42, comprend 350, 400, 500, ou 1000 acides aminés au maximum.

Dans un mode de réalisation préféré, les polypeptides sont constitués d'une séquence sélectionnée dans le groupe constitué de SEQ ID Nos. 1, 6, 9, 11, 18, 20, 24, 25, 26, 28, 29, 30, 31, 32, 36, 37, 40, 41 et 42 fusionnée à une étiquette de purification.

Le pourcentage d'identité selon l'invention peut être calculé par de nombreuses méthodes bien connues de l'homme du métier. De préférence, le pourcentage d'identité correspond au nombre d'acides aminés identiques obtenus pour un alignement apparié optimal (c'est-à-dire l'alignement maximisant le nombre d'acides aminés identiques) d'une séquence d'une protéine homologue avec SEQ ID Nos. 1, 6, 9, 11, 18, 20, 24, 25, 26, 28, 29, 30, 31, 32, 36, 37, 40, 41 et 42, sur la totalité de leur longueur, divisé par le nombre total d'acides aminés de la plus grande des deux séquences alignées. L'alignement peut être réalisé manuellement ou à l'aide de programmes d'ordinateur tels que le programme EMBOSS-Needle program (Needleman & Wunsch (1970) J. Mol. Biol. 48:443-453). Le pourcentage d'identité est de préférence calculé en utilisant le programme EMBOSS::needle (global) avec la matrice Blosum62 et les paramètres suivants: "Gap Open" égal à 10.0, "Gap Extend" égal à 0.5. De préférence, le pourcentage d'identité selon l'invention est d'au moins 85%, plus préférablement d'au moins 90%, et encore plus préférablement d'au moins 95%.

De préférence, le « fragment » selon l'invention comprend au moins 20 acides aminés, plus préférablement au moins 30 acides aminés, et encore plus préférablement au moins 40 acides aminés. De préférence également, le « fragment » selon l'invention comprend 100 acides aminés au plus, plus préférablement 80 acides aminés au plus, et encore plus préférablement 60 acides aminés au plus. Le fragments selon l'invention peut par exemple correspondre à la forme mature de la protéine (i.e. sans peptide signal). Ce fragment peut en outre être fusionné à d'autres séquences, telles qu'un peptide signal hétérologue et/ou une étiquette de purification.

### Méthodes

Les patients infectés par *Staphylococcus* produisent des anticorps contre des antigènes de *Staphylococcus.* De ce fait, les antigènes identifiés par les inventeurs peuvent être utilisés dans le cadre du sérodiagnostic d'infections à staphylocoques.

La présent demande décrit une méthode *in vitro* pour déterminer si un individu est infecté par une bactérie du genre *Staphylococcus*, de préférence *S*. *aureus,* comprenant la détection d'anticorps dirigés contre au moins une protéine comprenant ou constituée d'une séquence choisie parmi SEQ ID Nos. 1, 6, 9, 11, 18, 20, 24, 25, 26, 28, 29, 30, 31, 32, 36, 37, 40, 41 et 42, dans un échantillon biologique de l'individu.

Ainsi, est prévue une méthode pour déterminer si un individu est infecté par une bactérie du genre *Staphylococcus*, de préférence *S*. *aureus,* comprenant:
a) déterminer si des anticorps dirigés contre au moins une protéine comprenant ou constituée d'une séquence choisie parmi SEQ ID Nos. 1, 6, 9, 11, 18, 20, 24, 25, 26, 28, 29, 30, 31, 32, 36, 37, 40, 41 et 42 sont présents dans l'échantillon biologique de l'individu; et
b) en déduire si ledit individu souffre d'une infection par une bactérie du genre *Staphylococcus:*

De préférence, les anticorps détectés dans les échantillons biologiques selon l'invention sont des IgG.

Comme cela apparaîtra clairement à l'homme du métier, « un anticorps dirigé contre au moins une protéine comprenant ou constituée d'une séquence sélectionnée dans le groupe constitué de SEQ ID Nos. 1, 6, 9, 11, 18, 20, 24, 25, 26, 28, 29, 30, 31, 32, 36, 37, 40, 41 et 42 » vise tout anticorps de l'individu capable de reconnaître une protéine constituée de SEQ ID Nos. 1, 6, 9, 11, 18, 20, 24, 25, 26, 28, 29, 30, 31, 32, 36, 37, 40, 41 et 42, c'est-à-dire un anticorps spécifique de cette protéine, mais qui peut également reconnaître tout polypeptide tel que défini ci-dessus, notamment la forme mature de cette protéine.

S'agissant de la détection d'anticorps dirigés contre une protéine comprenant ou constituée de SEQ ID Nos. 1, 6, 9, 11, 18, 20, 24, 25, 26, 28, 29, 30, 31, 32, 36, 37, 40, 41 et 42 dans les échantillons biologiques, elle peut être réalisée à l'aide des polypeptides tels que définis ci-dessus. Ces polypeptides constituent alors des ligands de capture des anticorps présents dans l'échantillon biologique de l'individu.

Ainsi, l'étape (a) de détermination de la présence ou de l'absence d'anticorps dirigés contre au moins une protéine comprenant ou constituée d'une séquence choisie parmi SEQ ID Nos. 1, 6, 9, 11, 18, 20, 24, 25, 26, 28, 29, 30, 31, 32, 36, 37, 40, 41 et 42 dans l'échantillon biologique de l'individu peut comprendre les étapes de :
a₁) mettre l'échantillon biologique en contact avec au moins un polypeptide tel que défini ci-dessus ; et
a₂) détecter la présence ou l'absence d'anticorps liés au polypeptide, par exemple à l'aide d'un ligand de détection.

Dans un mode de réalisation préféré, la détection de la présence ou l'absence d'anticorps liés au polypeptide selon l'invention est réalisée par ELISA.

Un mode de réalisation particulier de l'invention concerne une méthode dans laquelle, en outre, des anticorps dirigés contre une protéine comprenant ou constituée de la séquence SEQ ID NO: 6 sont déterminés dans un échantillon biologique de l'individu. Cet anticorps est uniquement détecté chez la souche Mu50 (SARM) et non détecté chez la souche SASM étudiée. De ce fait, la détermination que des anticorps dirigés contre une protéine comprenant une séquence SEQ ID NO: NO: 6 sont présents dans l'échantillon biologique de l'individu indique que cet individu est infecté par une bactérie du genre *Staphylococcus*, en particulier *S. aureus,* qui est résistante à la méthicilline.

La présente demande décrit également une méthode *in vitro* pour déterminer si un individu est infecté par une bactérie du genre *Staphylococcus,* de préférence *S*. *aureus,* comprenant la détection d'une protéine comprenant ou constituée d'une séquence choisie parmi SEQ ID Nos. 1, 6, 9, 11, 18, 20, 24, 25, 26, 28, 29, 30, 31, 32, 36, 37, 40, 41 et 42 (i.e. la détection de l'antigène en tant que tel) dans un échantillon biologique de l'individu.

Dans ce cas, la détection d'antigènes de *Staphylococcus* peut être réalisée à l'aide d'un ligand de capture dirigé, de préférence spécifiquement, contre une protéine représentée par SEQ ID Nos. 1, 6, 9, 11, 18, 20, 24, 25, 26, 28, 29, 30, 31, 32, 36, 37, 40, 41 ou 42. Ce ligand de capture peut être de tout type, mais on préfère qu'il soit un anticorps, un aptamère, ou un peptide obtenu par « phage display », et en particulier on préfère qu'il soit un anticorps.

Pour déterminer si des anticorps ou des antigènes présents dans l'échantillon biologique ont été fixés par un ligand de capture on peut utiliser un ligand de détection, qui peut être spécifique soit des anticorps ou des antigènes fixés, soit des ligands de capture. Le ligand de détection peut être de tout type, mais on préfère qu'il soit un anticorps, un aptamère, ou un peptide obtenu par « phage display », et en particulier on préfère qu'il soit un anticorps. Ainsi, le ligand de détection peut par exemple correspondre à un anticorps anti-IgG, visant à détecter les anticorps de l'individu liés au polypeptide selon l'invention utilisé en tant que ligand de capture.

De préférence, le ligand de détection est marqué. Comme on l'entend ici, le terme « marqué » se réfère à la fois à un marquage direct et à un marquage indirect (par exemple par le biais d'un autre ligand lui-même marqué directement). Le marquage peut être de type radioisotopique, enzymatique ou lumineux (par exemple à l'aide d'un chromophore ou d'un fluorophore).

Les méthodes faisant appel à des ligands de capture et des ligands de détection sont bien connues de l'homme du métier et peuvent être mises en oeuvre selon différents formats bien connus, en phase solide ou homogène, en une ou deux étapes, à l'aide d'une méthode sandwich ou par compétition. De préférence, le ligand de capture est immobilisé sur une phase solide, telle que les parois d'un puits d'une plaque de microtitration ou des billes paramagnétiques.

### Kits

Est décrit un kit pour diagnostiquer une infection par une bactérie du genre *Staphylococcus, de* préférence *S*. *aureus,* comprenant au moins ou deux polypeptides selon l'invention tels que définis ci-dessus.

On décrit également un kit pour diagnostiquer une infection par une bactérie du genre *Staphylococcus,* de préférence S. *aureus,* comprenant au moins un ligand de capture dirigé, de préférence spécifiquement, contre une protéine représentée par SEQ ID Nos. 1, 6, 9, 11, 18, 20, 24, 25, 26, 28, 29, 30, 31, 32, 36, 37, 40, 41 ou 42. Ce ligand de capture peut être de tout type, mais on préfère qu'il soit un anticorps, un aptamère, ou un peptide obtenu par « phage display », et en particulier on préfère qu'il soit un anticorps.

Les kits peuvent par exemple contenir :
- un polypeptide selon l'invention et un ligand de détection permettant la détection d'anticorps liés au polypeptide selon l'invention (e.g. un anticorps anti-IgG, de préférence marqué) ; ou
- un ligand de capture dirigé, de préférence spécifiquement, contre une protéine représentée par SEQ ID Nos. 1, 6, 9, 11, 18, 20, 24, 25, 26, 28, 29, 30, 31, 32, 36, 37, 40, 41, ou 42 et un ligand de détection permettant la détection d'antigènes liés au ligand de capture.

Ces kits peuvent également comprendre des réactifs permettant de mettre en oeuvre la méthode selon l'invention et/ou des instructions d'utilisation.

### Utilisations

La présente demande concerne également l'utilisation pour le diagnostic *in vitro* d'une infection par une bactérie du genre *Staphylococcus,* en particulier *S*. *aureus* :
- d'un polypeptide selon l'invention défini ci-dessus ; ou
- d'un ligand de capture dirigé, de préférence spécifiquement, contre une protéine représentée par SEQ ID Nos. 1, 6, 9, 11, 18, 20, 24, 25, 26, 28, 29, 30, 31, 32, 36, 37, 40, 41 ou 42.

On décrit également l'utilisation pour déterminer *in vitro* si un individu souffre d'une infection par une bactérie du genre *Staphylococcus,* en particulier *S*. *aureus,* résistante à la méthicilline :
i. d'une protéine comprenant ou constituée d'une séquence SEQ ID NO: 6; ou
ii. d'une protéine homologue comprenant ou constituée d'une séquence partageant au moins 80%, 85%, 90%, 95%, 96%, 97%, 98% ou 99% d'identité avec une séquence SEQ ID NO: 6; ou
iii. d'une protéine comprenant ou constituée d'un fragment de la protéine définie en (i) ou en (ii), ledit fragment comprenant au moins 10 acides aminés consécutifs de ladite protéine définie en (i) ou en (ii); ou
iv. d'un ligand de capture dirigé, de préférence spécifiquement, contre une protéine représentée par SEQ ID NO : 6 ;
sous réserve que la protéine définie en (ii) ou que le fragment défini en (iii) puisse être lié par au moins anticorps dirigé contre une protéine comprenant ou constituée d'une séquence SEQ ID NO: 6.

Tous les articles, revues, demandes de brevet, brevets et manuels mentionnés ici sont incorporées par référence au texte de la présente demande.

Bien qu'ayant des significations distinctes, les termes « comprenant », « contenant », « comportant » et « consistant en » ont été utilisés de manière interchangeable dans la description de l'invention, et peuvent être remplacés l'un par l'autre.

Par ailleurs, l'invention sera davantage explicitée à l'aide des figures et exemples qui suivent.

### Description des figures

Figure 1 : Principe de l'immunoprotéome. PMF : Peptide Mass Fingerprinting, MS/MS : spectrométrie de masse en tandem.
Figure 2 : Analyse par immunoprotéome des protéines sécrétées par la souche Mu50 (SARM) avec 10 sérums de patients atteints d'infection sur prothèse à SARM (2A Gel 2D coloré au bleu de Coomassie et 2B immunoblot correspondant).
Figure 3 : Répartition et fonctions des antigènes identifiés.

### EXEMPLES

### Exemple 1 : Matériels et méthodes

### 1.1. Panel de patients

Vingt patients (adultes de 28 à 84 ans) souffrant d'IPA et admis entre 2004 et 2006 à l'Hôpital Raymond Poincaré (Garches) ou à l'Hôpital de la Croix Saint-Simon (Paris) ont été sélectionnés sur le critère d'au moins 3 prélèvements positifs par culture de *Staphylococcus aureus* résistant ou sensible à la méthicilline.

### 1.2. Souches, conditions de culture, et extraction protéique

Les souches Mu50 (SARM) et 29213 (SASM) ont été cultivées dans 100 mL de milieu TCS (Tryptic soy broth) à 37°C et sous agitation (200 rpm) pendant 6 heures. Le surnageant de culture a été concentré puis 10% de TCA (acide trichloroacétique) ont été ajoutés afin de précipiter les protéines correspondant à la fraction sécrétée du protéome (fraction Sc).

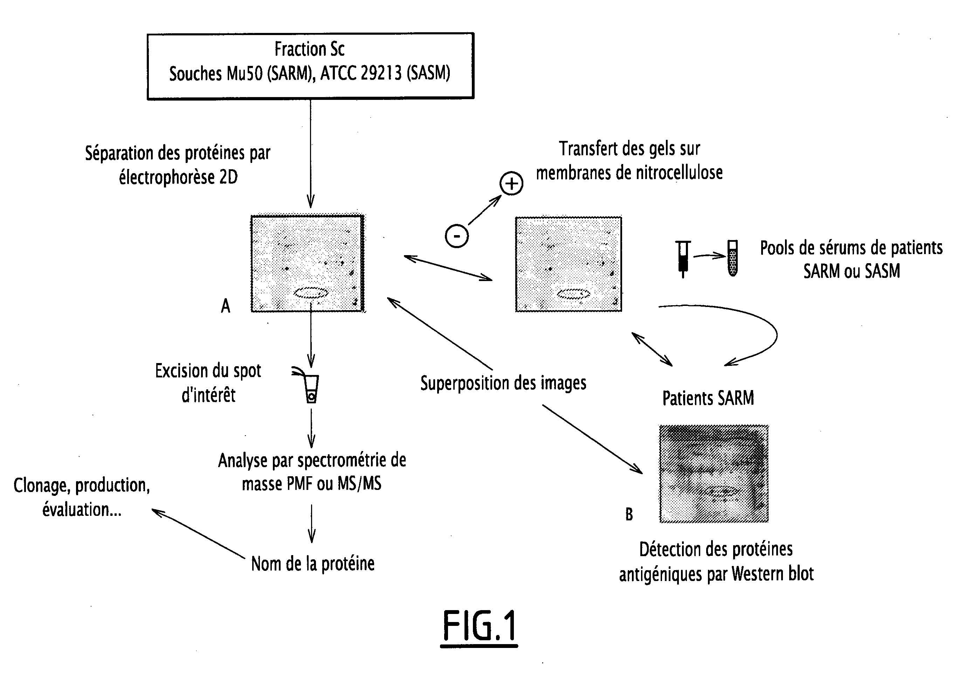

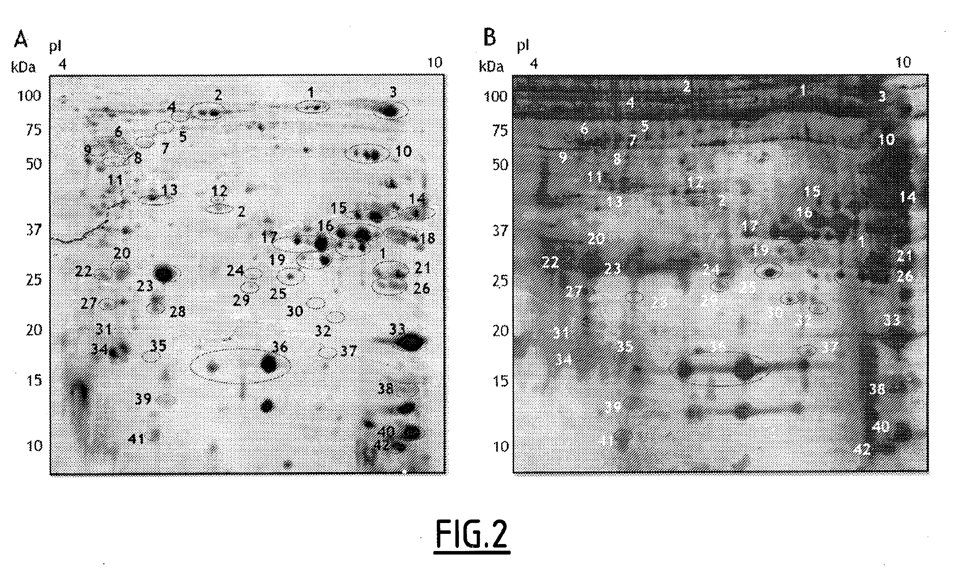

### 1.3. Analyse immunoprotéomique (Figures 1 et 2)

Après lavage à l'acétone glaciale, les protéines de la fraction Sc ont été solubilisées dans un tampon 2D (urée 7M, thiourée 2M, ASB-14 1%, Tris 40 mM). Une quantité de 500 µg de protéines est séparée par électrophorèse 2D de gamme de pH 3 à 10. Un gel est coloré au bleu de Coomassie (A), l'autre est transféré sur une membrane de nitrocellulose pour la révélation des antigènes en Western blot avec les sérums de criblage (B). Les différents antigènes indiqués au niveau du Western blot (B) par un numéro sont localisés de la même façon au niveau du gel coloré au bleu de Coomassie (A). Les protéines antigéniques ont ensuite été identifiées par spectrométrie de masse (PMF et/ou MS/MS) chez la souche Mu50.

### 1.4. Sérodiagnostic des IPOA par test ELISA

Les plaques ELISA sont laissées pendant une nuit à 4°C en présence de 0,5 µg d'antigène purifié dans du « tampon salin phosphate » (PBS). Après quatre lavages par du PBS contenant du polyoxyéthylène sorbitan (Tween) à 0,05%, les plaques sont saturées une heure et demie à 37°C par du PBS-Tween contenant 4% d'albumine de sérum bovin (BSA) (250 µl par puits). Quatre nouveaux lavages sont réalisés puis 100 µl de chaque sérum « positif » ou témoin sont ajoutés, à la dilution 1/100 dans du tampon PBS-BSA 4%, dans chaque puits. La plaque est ensuite incubée à 37°C pendant 30 minutes. Après quatre nouveaux lavages, des anticorps anti-immunoglobulines G humaines de chèvre marqués à la peroxydase (par exemple 31415, Pierce) sont ajoutés (simultanément et/ou indépendamment) en 30 minutes à 37°C après avoir été dilués, selon le protocole du fournisseur, dans du tampon PBS-BSA 4%. Quatre nouveaux lavages sont réalisés puis 100 µl de substrat tétrabenzimidine (TMB) (par exemple HD979505, Pierce) sont ajoutés par puits. Après incubation pendant environ 15 minutes, 100 µl d'acide sulfurique sont ajoutés dans chaque puits. L'absorbance à 450 nm de chaque puits est alors mesurée après 5 minutes. Sont considérés comme « positif » en ELISA, les sérums identifiés par leur liaison avec les polypeptides (antigènes) tels que définis selon l'invention. Les performances des tests ELISA utilisant les polypeptides identifiés selon l'invention ainsi que les combinaisons de polypeptides selon l'invention ont été évaluées par la méthode d'analyse par fonction discriminante, analyse très proche de l'analyse de variance. Cette méthode connue de l'homme du métier permet de déterminer les variables qui permettent de déterminer entre les deux groupes de patients ou individus témoins. La méthode consiste à déterminer si les deux groupes diffèrent suivant la moyenne de la variable et d'utiliser cette dernière pour prédire le groupe.

### Exemple 2 : Résultats

Parmi l'ensemble des protéines sécrétées par la bactérie dans le surnageant de culture, 41 protéines antigéniques ont pu être identifiées chez les deux souches étudiées. Un antigène (SEQ ID NO : 6) n'est sécrété que par la souche SARM étudiée (Mu50).

La séquence de ces quarante deux antigènes est présentée ci-dessous.
SEQ ID NO: 1 (*S*. *aureus*)
SEQ ID NO: 2 (*S*. *aureus*)
SEQ ID NO: 3 (*S. aureus)*
SEQ ID NO: 4 (*S. aureus*)
SEQ ID NO: 5 (*S. aureus*)
SEQ ID NO: 6 (*S*. *aureus*)
SEQ ID NO: 7 (S. *aureus)*
SEQ ID NO: 8 (*S. aureus*)
SEQ ID NO: 9 (*S*. *aureus*)
SEQ ID NO: 10 (*S*. *aureus*)
SEQ ID NO: 11 (*S. aureus*)
SEQ ID NO: 12 (*S*. *aureus*)
SEQ ID NO: 13 (*S*. *aureus*)
SEQ ID NO: 14 (*S. aureus*)
SEQ ID NO: 15 (*S*. *aureus*)
SEQ ID NO: 16 (*S*. *aureus*)
SEQ ID NO: 17 (*S*. *aureus*)
SEQ ID NO: 18 (*S*. *aureus*)
SEQ ID NO: 19 (*S*. *aureus*)
SEQ ID NO: 20 (*S. aureus*)
SEQ ID NO: 21 (*S. aureus*)
SEQ ID NO: 22 (*S. aureus*)
SEQ ID NO: 23 (*S*. *aureus*)
SEQ ID NO: 24 (*S. aureus*)
SEQ ID NO: 25 (*S. aureus*)
SEQ ID NO: 26 (*S*. *aureus*)
SEQ ID NO: 27 (*S. aureus*)
SEQ ID NO: 28 (*S. aureus*)
SEQ ID NO: 29 (*S. aureus*)
SEQ ID NO: 30 (*S. aureus*)
SEQ ID NO: 31 (*S. aureus*)
SEQ ID NO: 32 (*S. aureus*)
SEQ ID NO: 33 (*S*. *aureus*)
SEQ ID NO: 34 (*S. aureus*)
SEQ ID NO: 35 (*S*. *aureus*)
SEQ ID NO: 36 (*S. aureus*)
SEQ ID NO: 37 (*S. aureus*)
SEQ ID NO: 38 (*S. aureus*)
SEQ ID NO: 39 (*S. aureus*)
SEQ ID NO: 40 (*S. aureus*)
SEQ ID NO: 41 (*S*. *aureus*)
SEQ ID NO: 42 (*S. aureus*)

La sécrétion de 40 des 42 antigènes a été confirmée par la présence de séquences caractéristiques identifiées *in silico.* Sur ces 40 antigènes, 9 protéines (celles de séquences SEQ ID NO: 6, 9, 18, 20, 29, 30, 33, 36 et 37) n'avaient pas encore été détectées dans le surnageant de culture de staphylocoque auparavant (Royan et al. 2000. FEMS Immunol Med Microbiol. 29:315-21). Deux protéines ne possèdent pas de séquence de sécrétion connue.

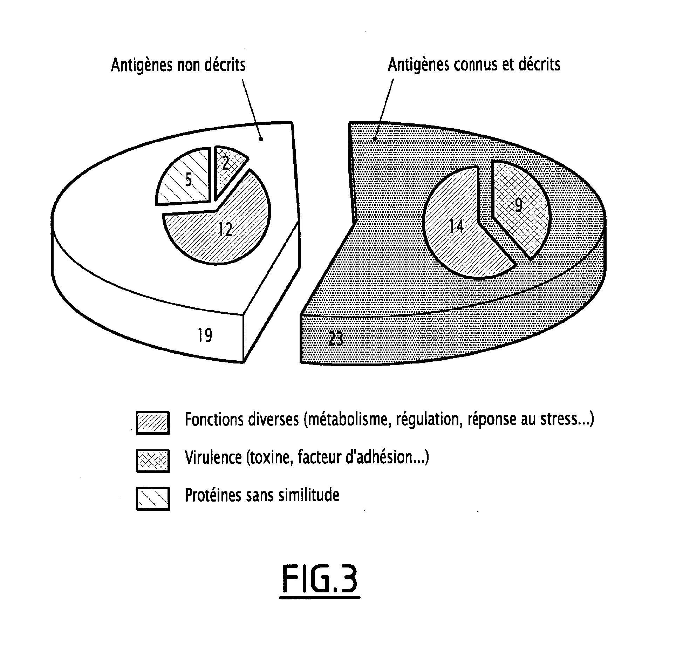

Parmi les 42 protéines antigéniques identifiées lors de notre étude, 23 antigènes, tels que l'α hémolysine, l'entérotoxine P ou encore les antigènes immunodominants IsaA ou IsaB, avaient déjà été décrits dans la littérature (voir tableau 1 et Figure 3).

**Tableau 1 - Caractérisation des antigènes identifiés**

| **SEQ ID NO :** | **Nom de la protéine** | **N°gène** | **Sécrétion* (publications)** | **Antigénicité : publications** |
|---|---|---|---|---|
| 2 | Autolysine | SAV1052 | Oui* (2, 9) | 1,2 |
| 3 | Lipase 2 geh | SAV320 | Oui* (2, 9) | 2 |
| 4 | Staphylocoagulase | SAV0230 | Oui* (2) | 2 |
| 5 | Inositol monophosphate dehydrogenase | SAV0390 | Oui* (2, 9) | 1,2 |
| 7 | 6 phosphogluconate dehydrogenase | SAV1511 | Oui* (2) | 2 |
| 8 | Protéine A | SAV0111 | Oui* (2) | 2,5 |
| 10 | Anion binding protein | SAV0719 | Oui* (2, 9) | 2 |
| 12 | Alcohol dehydrogenase | SAV0605 | Oui* (2) | 1 |
| 13 | Cysteine protease precursor | SAV1047 | Oui* (2, 9) | 2 |
| 14 | γ hemolysin component C | SAV2420 | Oui* (9) | 7 |
| 15 | γ hemolysi chain II | SAV2419 | Oui* (9) | 7 |
| 16 | α Hemolysi chain A | SAV1163 | Oui* (2, 9) | 4 |
| 17 | 1 phosphatidyl inositol phosphodiestérase precursor | SAV0095 | Oui* (2, 9) | 2 |
| 19 | Enterotoxin P | SAV1948 | Oui* (9) | 6 |
| 21 | V8 protease | SAV1048 | Oui* (2, 9) | 3 |
| 22 | Triose phosphate isomerase | SAV0774 | Oui* (2) | 1,2 |
| 23 | Immunodominant antigen A | SAV2569 | Oui* (2, 9) | 8, 12 |
| 27 | Alkyl hydroperoxyde reductase subunit C | SAV0381 | Oui* (9) | 1,2 |
| 33 | Microccal nuclease | SAV0815 | | 11 |
| 34 | Alkaline shock protein 23 | SAV2182 | Oui* (2, 9) | 2 |
| 35 | Universal stress family protein | SAV1710 | Oui* (9) | 1 |
| 38 | Immunodominant antigen B | SAV2638 | Oui* (9) | 8 |
| 39 | Staphylokinase precursor (^{f}) | SAV 1944 | Oui* (2) | 2 |

| | | | | |
|---|---|---|---|---|
| Légende du tableau 1 : (^{f}) identification d'un fragment de la protéine, (*) calcul du score extracellulaire par les logiciels PSORT2b (par exemple disponible sur le site internet psort.org/psortb), Signal P (par exemple disponible sur le site internet cbs.dtu.dk/services/SignalP/) et/ou Secretome P pour la voie de sécrétion non classique (par exemple disponible sur le site internet cbs.dtu.dk/services/SecretomeP/), puis confirmation éventuelle dans la littérature. | | | | |

La présente étude a permis d'identifier 19 nouveaux antigènes, encore jamais identifiés comme tels (voir tableau 2 et Figure 3). Cinq d'entre eux sont décrits comme étant des facteurs de virulence. Les douze autres possèdent des fonctions très variées et sont impliqués dans le métabolisme de la bactérie, le transport de métabolite ou encore dans la régulation de l'expression de gènes. Enfin, les deux derniers n'ont encore jamais été étudiés et ne présentent pas d'homologies permettant de leur attribuer une fonction.

**Tableau 2 - Caractérisation des antigènes identifiés**

| **SEQ ID NO:** | **Nom de la protéine** | **N° gène** | **Sécrétion* (publications)** | **Fonction ou fonction putative** | **Similitude avec des protéines de fonction connue, remarques** |
|---|---|---|---|---|---|
| 1 | Glycérophosphodiester phosphodiestérase | SAV959 | Oui* (9) | Métabolisme phospholipidique | |
| 6 | Hypothetical protein | SAV434 | Oui* | Inconnue | Pas d'homologie avec des protéines aux fonctions décrites, antigénique chez SARM uniquement |
| 9 | Hypothetical protein | SAV2032 | Oui* | Adhésion | Ser-Asp rich fibrinogen/bone sialoprotein bindin protein. Similaire à SdrH de S. aureus. MW2 : 105/415 => 25% (100% d'homologie sur les 105 communs) |
| 11 | Sugar efflux transporter | SAV0673 | Oui* (2, 9) | Impliqué dans le transport de sucres | |
| 18 | Leucoccidine non caratérisée (chez Mu50) | SAV2004 | Oui* | Trouble de la réponse immunitaire Lyse des leucocytes (polynucléaires neutrophile) | 100% d'idendité avec Luk F de S. aureus (souche RF122) |
| 20 | Probable transglycosylase SceD | SAV2095 | Oui* | Impliquée dans le clivage du peptidoglycane | |
| 24 | N acetyl muramoyl L alanine amidase (^{f}) | SAV2307 | Oui* (9) | Catabolisme de la paroi | |
| 25 | Serine protease SpIC | SAV1811 | Oui* (9) | Activité protéasique (trypsine « like ») | |
| 26 | Serine protease SpIF | SAV1809 | Oui* (9) | Activité protéasique (trypsine « like ») | |
| 28 | Hypothetical protein | SAV0372 | Oui* (2, 9) | Inconnue | Lipoprotein de S. aureus (souche MN8) 98% |
| 29 | Exotoxin 15 superantigène « like » de S. aureus | SAV0433 | Oui* | Troubles de la réponse immunitaire Hyper stimulation des lymphocytes T | |
| 30 | Exotoxin 6 superantigène « like » de S. aureus | SAV0422 | Oui* | | |
| 31 | Hypothetical protein | SAV0171 | Non | Transduction du signal | Alpha-helical coiled-coil protein Microcystis aeruginosa NIES-843 :44% |
| 32 | Hypothetical protein | SAV0613 | Oui* (9) | Inconnue | |
| 36 | Hypothetical protein | SAV2205 | Oui* | Adhésion, trouble de la réponse immunitaire | Analogue du complexe majeur d'histocompatibilité (MAP domain) Extracellular adherence protein like protein de S. aureus (souche MN8) : 99% |
| 37 | Hypothetical protein | SAV0814 | Oui* | Adhésion aux matrices du plasma Homéostasie | Homologue tronqué du facteur (sécrété) de von Willebrand de S. aureus (souche RF122) :85% 147/499 (29%) sur la globalité du gène |
| 40 | Hypothetical protein | SAV0981 | Oui* (9) | Adhésion, trouble de la réponse immunitaire | Analogue du complexe majeur d'histocompatibilité Extracellular adherence protein Eap de S. aureus (souche MN8) :143/144 (99%) |
| 41 | Hypothetical protein | SAV1942 | Oui* (2) | Adhésion, trouble de la réponse immunitaire | SCIN (Staphylococcal Complement Inhibitor) de S. aureus (souche MRSA252) (69%) Fibrinogen-binding protein de S. aureus (souche MN8) : 49% |
| 42 | Staphylococcal respiratory response A ssrA, (^{f}) de S. aureus | SAV1492 | Non | Régulateur transcriptionnel pléiotropique (système Agr, protéine A, TSST-1) | |

| | | | | | |
|---|---|---|---|---|---|
| Légende du tableau 2 : (^{f}) identification d'un fragment de la protéine, (*) calcul du score extracellulaire par les logiciels PSORT2b (par exemple disponible sur le site internet psort.org/psortb), Signal P (par exemple disponible sur le site internet cbs.dtu.dk/services/SignalP/) et/ou Secretome P pour la voie de sécrétion non classique (par exemple disponible sur le site internet cbs.dtu.dk/services/SecretomeP/), puis confirmation éventuelle dans la littérature. | | | | | |

Les performances de plusieurs des nouveaux antigènes identifiés pour le sérodiagnostic des IPOA à *S*. *aureus* ont été étudiées par test ELISA en utilisant des panels de 18 sérums de patients infectés et de 79 sérums de donneurs de sang témoins (tableau 3).

**Tableau 3 : Exemples de résultats (ELISA) en utilisant des anti-IgG comme anticorps secondaires**

| Polypeptides testés | Proportion de sérums « positifs » parmi les 18 sérums de patients infectés par *S*. *aureus* et diagnostiqués par culture | Proportion de sérums « négatifs » parmi 79 sérums témoins (donneurs de sang). |
|---|---|---|
| SEQ ID NO: 1 | 55,6% (10/18) | 91,1% (72/79) |
| SEQ ID NO: 18 | 55,6% (10/18) | |
| SEQ ID NO: 24 | 66,7% (12/18) | |
| SEQ ID NO: 40 | 33,3% (6/18) | |
| SEQ ID NO: 42 | 50% (9/18) | |
| SEQ ID NO: 17 | 5,6% (1/18) | 91,1% (72/79) |

Il ressort de ces résultats que les polypeptides de séquence SEQ ID NO: 18, SEQ ID NO: 40, SEQ ID NO: 24, SEQ ID NO: 1 et SEQ ID NO: 42 permettent de discriminer les patients des témoins alors que le polypeptide SEQ ID NO: 17, bien que déjà décrit comme antigénique dans la littérature, ne le permet pas.

En conclusion, cette étude a permis d'identifier 19 nouveaux antigènes candidats au développement d'un test de sérodiagnostic d'IPOA.

La production sous forme recombinante et la purification de ces antigènes sont en cours. Les performances diagnostiques (sensibilité, spécificité) des antigènes sont évaluées dans la perspective du développement d'un test multiparamétrique de sérodiagnostic des IPOA. Une étude clinique multicentrique française incluant 5 centres (Croix St Simon, Berck sur Mer, Lyon, Strasbourg, Garches-Amboise Paré [UTL], etc.) a été initiée afin de récolter des sérums de patients atteints d'IPOA. Le gène codant l'antigène 6, uniquement détecté chez la souche Mu50 (SARM) et non détecté chez la souche SASM étudiée est séquencé chez plusieurs souches SARM et SASM. Des études sont menées afin de confirmer l'intérêt de cette protéine pour le diagnostic des infections à SARM versus SASM.

### Références bibliographiques

1. Brady, R. A., J. G. Leid, A. K. Camper, R. W. Costerton, and M. E. Shirlift. 2006. Identification of Staphylococcus aureus proteins recognized by the antibody-mediated immune response to a biofilm infection. Infect. Immun. 74:3415-26.
2. Burlak C., C. H. Hammer, M. A. Robinson, A. R. Whitney, M. J. McGavin, B. N. Kreiswirth and F. R. Deleo. 2007. Global analysis of community-associated methicillin-resistant Staphylococcus aureus exoproteins reveals molecules produced in vitro and during infection. Cell Microbiol. 9:1172-90.
3. Calander A. M., G. Dubin, J. Potempa, A. Tarkowski. 2008. Staphylococcus aureus infection triggers production of neutralizing, V8 protease-specific antibodies. FEMS Immunol Med Microbiol. 52:267-72.
4. Christensson B., S. A. Hedström, and G. Kronvall. 1983. Antibody response to alpha- and betahemolysin from Staphylococcus aureus in patients with staphylococcal infections and in normals. Acta Pathol Microbiol Immunol Scand. 91:351-6. Graille M., E. A. Stura, A. L . Corper, B. J. Sutton, M. J. Taussig, J. B. Charbonnier, and G. J. Silverman. 2000. Crystal structure of a Staphylococcus aureus protein A domain complexed with the Fab fragment of a human IgM antibody: structural basis for récognition of B-cell receptors and superantigen activity. Proc Natl Acad Sci USA. 97:5399-404.
5. Gruber J., G. C. Wright. 1969. Ammonium sulfate coprecipitation antibody determination with purified staphylococcal enterotoxins. J Bacteriol. 99:18-24.
6. Kurek M., K. Pryjma, S. Bartkowski, and P. B. Heczko. 1977. Anti-staphylococcal gamma hemolysin antibodies in rabbits with staphylococcal osteomyelitis. Med Microbiol Immunol. 163:61-5.
7. Lorenz U., K. Ohlsen, H. Karch, M. Hecker, A. Thiede, and J. Hacker. 2000. Human antibody response during sepsis against targets expressed by methicillin resistant Staphylococcus aureus. FEMS Immunol Med Microbiol. 29:145-53.
8. Pocsfalvi G., G. Cacace, M. Cuccurullo, G. Serluca, A. Sorrentino, G. Schlosser, G. Blaiotta, and A. Malorni. 2008. Proteomic analysis of exoproteins expressed by enterotoxigenic Staphylococcus aureus strains. Proteomics. 8:2462-76.
9. Royan S., L. Sharp, S. P. Nair, S. Crean, B. Henderson, S. Poole, G. L. Scott, and A. W. Evans. 2000. Identification of the secreted macromolecular immunogens of Staphylococcus aureus by analysis of serum. FEMS Immunol Med Microbiol. 29:315-21.
10. Verbrugh H. A. , R. D. Nelson, P. K. Peterson, B. J. Wilkinson, and R. L. Thompson. 1983. Serology of Staphylococcus aureus infections using multiple antigens and serial serum samples. J Infect Dis. 148:608.
11. Vytvytska O., E. Nagy, M. Blüggel, H. E. Meyer, R. Kurzbauer, L. A. Huber, and C. S. Klade. 2002. Identification of vaccine candidate antigens of Staphylococcus aureus by serological proteome analysis. Proteomics. 2:580-90.

## Revendications

1. Méthode *in vitro* pour déterminer si un individu est infecté par une bactérie du genre *Staphylococcus,* comprenant la détection d'anticorps dirigés contre au moins une protéine comprenant une séquence SEQ ID NO: 40, dans un échantillon biologique de l'individu.

2. Méthode selon la revendication 1 comprenant les étapes de:
a) déterminer si des anticorps dirigés contre au moins une protéine comprenant la séquence SEQ ID NO: 40 sont présents dans l'échantillon biologique de l'individu; et
b) en déduire si ledit individu souffre d'une infection par une bactérie du genre *Staphylococcus.*

3. Méthode selon la revendication 1 ou 2, dans laquelle la bactérie du genre *Staphylococcus* est *Staphylococcus aureus.*

4. Méthode selon l'une des revendications 1 à 3, dans laquelle l'individu a subi l'implantation d'une prothèse articulaire.

5. Méthode selon l'une des revendications 1 à 4, dans laquelle l'individu a subi l'implantation d'une prothèse articulaire sélectionnée dans le groupe constitué d'une prothèse du genou, une prothèse de hanche et une prothèse d'épaule.

6. Méthode selon l'une des revendications 1 à 5, dans laquelle l'échantillon biologique est sélectionné dans le groupe constitué d'un échantillon de sang, d'un échantillon de sérum, d'un échantillon de plasma, d'un échantillon de tissu lymphoïde associé à une muqueuse (MALT), un échantillon de liquide céphalo-rachidien, un échantillon de liquide articulaire, un échantillon de liquide pleural, un échantillon de salive, et un échantillon d'urine.

7. Méthode selon l'une des revendications 2 à 6, dans laquelle l'étape (a) de détermination de la présence ou de l'absence d'anticorps dirigés contre au moins une protéine comprenant la séquence SEQ ID NO: 40 dans l'échantillon biologique de l'individu comprend les étapes de :
a₁) mettre l'échantillon biologique en contact avec au moins:
(i) une protéine comprenant la séquence SEQ ID NO: 40; ou
(ii) une protéine comprenant une séquence partageant au moins 80% d'identité avec la séquence SEQ ID NO: 40 ou
(iii) un fragment de la protéine définie en (i) ou en (ii), ledit fragment comprenant au moins 10 acides aminés ;
sous réserve que la protéine définie en (ii) ou que le fragment défini en (iii) puisse être lié par au moins anticorps dirigé contre une protéine comprenant la séquence SEQ ID NO: 40 et
a₂) détecter la présence ou l'absence d'anticorps liés à la protéine ou au fragment
défini à l'étape a₁).

8. Méthode selon l'une des revendications 2 à 7, dans laquelle l'étape (a) comprend de plus, la détermination de la présence ou de l'absence d'anticorps dirigés contre une protéine comprenant une séquence SEQ ID NO: 6, et dans laquelle la détermination que des anticorps dirigés contre une protéine comprenant une séquence SEQ ID NO: NO: 6 sont présents dans l'échantillon biologique de l'individu indique que l'individu est infecté par une bactérie du genre *Staphylococcus* résistante à la méthicilline.

## Patentansprüche

1. In-Vitro-Verfahren zum Bestimmen, ob ein Individuum mit einem Bakterium der Gattung *Staphylococcus* infiziert ist, das die Detektion von Antikörpern, die gegen mindestens ein Protein mit einer Sequenz SEQ ID NR.: 40 gerichtet sind, in einer biologischen Probe des Individuums umfasst.

2. Verfahren nach Anspruch 1, das die Schritte umfasst:
a) Bestimmen, ob Antikörper, die gegen mindestens ein Protein mit der Sequenz SEQ ID NR.: 40 gerichtet sind, in der biologischen Probe des Individuums vorhanden sind; und
b) daraus Ableiten, ob das Individuum unter einer Infektion mit einem Bakterium der Gattung *Staphylococcus* leidet.

3. Verfahren nach Anspruch 1 oder 2, wobei das Bakterium der Gattung *Staphylococcus Staphylococcus aureus* ist.

4. Verfahren nach einem der Ansprüche 1 bis 3, wobei das Individuum einer Implantation einer Gelenkprothese unterzogen wurde.

5. Verfahren nach einem der Ansprüche 1 bis 4, wobei das Individuum einer Implantation einer Gelenkprothese unterzogen wurde, die aus der Gruppe ausgewählt ist, die aus einer Knieprothese, einer Hüftprothese und einer Schulterprothese besteht.

6. Verfahren nach einem der Ansprüche 1 bis 5, wobei die biologische Probe aus der Gruppe ausgewählt ist, die aus einer Blutprobe, einer Serumprobe, einer Plasmaprobe, einer Lymphgewebeprobe, die einer Schleimhaut (MALT) zugeordnet ist, einer Zerebrospinalflüssigkeitsprobe, einer Gelenkflüssigkeitsprobe, einer Rippenfellflüssigkeitsprobe, einer Speichelprobe und einer Urinprobe besteht.

7. Verfahren nach einem der Ansprüche 2 bis 6, wobei der Schritt (a) der Bestimmung der Anwesenheit oder der Abwesenheit von Antikörpern, die gegen mindestens ein Protein mit der Sequenz SEQ ID NR.: 40 gerichtet sind, in der biologischen Probe des Individuum die Schritte umfasst:
a₁) Kontaktieren der biologischen Probe mit mindestens:
(i) einem Protein mit der Sequenz SEQ ID NR.: 40; oder
(ii) einem Protein mit einer Sequenz, die sich mindestens 80 % Identität mit der Sequenz SEQ ID NR.: 40 teilt, oder
(iii) einem Fragment des in (i) oder in (ii) definierten Proteins, wobei das Fragment mindestens 10 Aminosäuren umfasst;
unter dem Vorbehalt, dass das in (ii) definierte Protein oder das in (iii) definierte Fragment durch mindestens einen Antikörper gebunden werden kann, der gegen ein Protein mit der Sequenz SEQ ID NR.: 40 gerichtet ist, und
a₂) Detektieren der Anwesenheit oder Abwesenheit von Antikörpern, die an das in Schritt a₁) definierte Protein oder Fragment gebunden sind.

8. Verfahren nach einem der Ansprüche 2 bis 7, wobei der Schritt (a) außerdem die Bestimmung der Anwesenheit oder Abwesenheit von Antikörpern umfasst, die gegen ein Protein mit einer Sequenz SEQ ID NR.: 6 gerichtet sind, und wobei die Bestimmung, dass Antikörper, die gegen ein Protein mit einer Sequenz SEQ ID NR.: 6 gerichtet sind, in der biologischen Probe des Individuums vorhanden sind, darauf hinweist, dass das Individuum mit einem Bakterium der Gattung *Staphylococcus,* das gegen Methicillin resistent ist, infiziert ist.

## Claims

1. An in vitro method for determining if an individual is infected by a bacterium of the genus *Staphylococcus* comprising the detection of antibodies directed against at least one protein comprising a sequence SEQ ID NO: 40 in a biological sample of an individual.

2. The method according to claim 1, comprising the steps of:
a) determining if antibodies directed against at least one protein comprising the sequence SEQ ID NO: 40 are present in a biological sample of the individual, and
b) deducing therefrom if the individual is infected by a bacterium of the genus *Staphylococcus.*

3. The method according to claim 1 or 2, wherein the bacterium of the genus *Staphylococcus* is *Staphylococcus aureus.*

4. The method according to any of claims 1 to 3, wherein the individual has been implanted a prosthetic joint.

5. The method according to any of claims 1 to 4, wherein the individual has been implanted a prosthetic joint selected from the group consisting of a knee joint, a hip joint and a shoulder joint.

6. The method according to any of claims 1 to 5, wherein the biological sample is selected from the group consisting of a blood sample, a serum sample, a plasma sample, a mucosa-associated lymphoid tissue (MALT) sample, a cerobrospinal fluid sample, an articular liquid sample, a pleural liquid sample, a saliva sample, and a urine sample.

7. The method according to any of claims 2 to 6, wherein the step a) of determining the presence or absence of antibodies directed against at least one protein comprising the sequence SEQ ID NO: 40 in the biological sample of the individual comprises the steps of:
a₁) contacting the biological sample with at least:
i) one protein comprising the sequence SEQ ID NO: 40, or
ii) one protein comprising a sequence sharing at least 80% identity with the sequence SEQ ID NO: 40; or
iii) one fragment of the protein defined in i) or in ii) wherein said fragment comprises at least 10 amino acids;
provided that the homologous protein defined in ii) or the fragment defined in iii) can be bound by at least one antibody directed against a protein comprising the sequence SEQ ID NO: 40; and
a₂) detecting the presence or absence of antibodies bound to the protein or fragment defined in step a₁).

8. The method according to any of claims 2 to 7, wherein the step a) comprises in addition, the determination of the presence or absence of antibodies directed against a protein comprising a sequence SEQ ID NO: 6, and wherein determining that antibodies directed against one protein comprising a sequence SEQ ID NO: 6 are present in a biological sample of the individual indicates that the individual is infected by a bacterium of the genus *Staphylococcus* resistant to methicillin.
